(19) Europäisches Patentamt
European Patent Office
Office européen des brevets

(11) Veröffentlichungsnummer: **0 072 960**
**B1**

(12) # EUROPÄISCHE PATENTSCHRIFT

(45) Veröffentlichungstag der Patentschrift: **07.06.89**

(21) Anmeldenummer: **82107193.3**

(22) Anmeldetag: **09.08.82**

(51) Int. Cl.⁴: **C 07 D 401/12,**
C 07 D 409/12,
C 07 D 231/22,
A 61 K 31/415, A 61 K 31/495

(54) **1,5-Diphenylpyrazolin-3-on-Verbindungen sowie Verfahren und Zwischenprodukte zu ihrer Herstellung und diese Verbindungen enthaltende Arzneimittel.**

(30) Priorität: **20.08.81 DE 3132915**

(43) Veröffentlichungstag der Anmeldung:
**02.03.83 Patentblatt 83/09**

(45) Bekanntmachung des Hinweises auf die Patenterteilung:
**07.06.89 Patentblatt 89/23**

(84) Benannte Vertragsstaaten:
**AT BE CH DE FR GB IT LI LU NL SE**

(56) Entgegenhaltungen:
**DE-A-2 824 764**
**DE-A-2 828 529**

**BEILSTEINS "Handbuch der organischen Chemie", 4. Auflage, Band 24 1936, VERLAG SPRINGER, Berlin Seiten 149-150 & ANNALEN DER CHEMIE, Band 358, Seite 159 (Kat. A, D)**

**Die Akte enthält technische Angaben, die nach dem Eingang der Anmeldung eingereicht wurden und die nicht in dieser Patentschrift enthalten sind.**

(73) Patentinhaber: **Kali-Chemie Pharma GmbH**
**Hans-Böckler-Allee 20 Postfach 220**
**D-3000 Hannover 1 (DE)**

(72) Erfinder: **Heinemann, Henning Dipl.-Chem., Dr.rer.nat.**
**Bergiusstrasse 18**
**D-3000 Hannover 51 (DE)**
Erfinder: **Jasserand, Daniel**
**2, allée d'Andrezieux**
**F-75018 Paris (FR)**
Erfinder: **Milkowski, Wolfgang Dipl.-Chem., Dr.rer.nat.**
**Fasanenweg 13**
**D-3167 Burgdorf (DE)**
Erfinder: **Yavordios, Dimitri**
**536, Av. Dubanchet**
**F-01400 Châtillon s/Chalaronne (FR)**
Erfinder: **Zeugner, Horst Dipl.-Chem. Dr.rer.nat.**
**Havelweg 10**
**D-3000 Hannover 73 (DE)**

(74) Vertreter: **Lauer, Dieter, Dr.**
**c/o Kali-Chemie AG Postfach 220 Hans-Böckler-Allee 20**
**D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 072 960 B1

Courier Press, Leamington Spa, England.

**Beschreibung**

Die vorliegende Erfindung betrifft neue 2-Piperazinoalkyl-1,5-diphenylpyrazolin-3-on-Verbindungen und deren Salze sowie diese Verbindungen enthaltende pharmazeutische Zubereitungen und Verfahren und Zwischenprodukte zur Herstellung dieser Verbindungen.

Der Erfindung liegt die Aufgabe zugrunde, neue 1,5-Diphenylpyrazolin-3-on-Verbindungen mit wertvollen pharmakologischen Eigenschaften sowie Verfahren und Zwischenprodukte zu ihrer Herstellung bereitzustellen.

Aus der DE—A—28 28 529 sind in 1-Stellung des Pyrazolinrestes durch Alkyl, Phenyl oder Benzyl substituierte 1-(5-Phenylpyrazol-3-oxy)-alkancarbonsäuren und deren Ester und Amide, welche als Hauptwirkung den Cholesterin- und Lipidgehalt des Blutes senkende Wirkung zeigen, bekannt.

Die erfindungsgemäßen 1,5-Diphenylpyrazolin-3-on-Verbindungen unterscheiden sich in ihrer chemischen Struktur wesentlich von den vorbekannten Pyrazolinderivaten durch die freie Ketofunktion in 3-Stellung und dadurch, daß sie an dem Stickstoff in 2-Position einen basischen Arylpiperazinoalkylrest tragen.

Es wurde nun gefunden, daß die neuen 1,5-Diphenylpyrazolin-3-on-Verbindungen wertvolle pharmakologische Eigenschaften, insbesondere ausgeprägte antiallergische Eigenschaften sowie daneben auch blutdrucksenkende Eigenschaften, besitzen und ein vorteilhaftes Wirkungsprofil mit guter therapeutischer Breite und geringer Toxizität aufweisen. Aufgrund dieser Eigenschaften sind die neuen Verbindungen als Arzneimittel zur Behandlung von allergischen Erkrankungen wie beispielsweise allergisch bedingtem Asthma oder Heuschnupfen geeignet.

Die vorliegende Erfindung betrifft daher neue 1,5-Diphenylpyrazolin-3-on-Verbindungen der allgemeinen Formel I

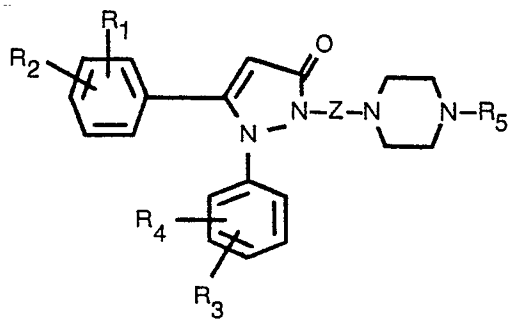

I

worin

$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_2$ Wasserstoff, Alkyl mit 1—4 Kohlenstoffatomen, Alkoxy mit 1—4 Kohlenstoffatomen oder Halogen bedeutet, oder

$R_1$ und $R_2$ an benachbarte Kohlenstoffatome gebunden sind und zusammen Methylendioxy oder Äthylendioxy bedeuten,

$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_4$ Wasserstoff, Alkyl mit 1—4 Kohlenstoffatomen, Alkoxy mit 1—4 Kohlenstoffatomen oder Halogen bedeutet, oder

$R_3$ und $R_4$ an benachbarte Kohlenstoffatome gebunden sind und zusammen Methylendioxy oder Äthylendioxy bedeuten,

Z eine Alkylengruppe mit 2—6 Kohlenstoffatomen bedeutet,

$R_5$ Pyridyl, welches unsubstituiert oder durch Alkyl mit 1—4 Kohlenstoffatomen, Halogen oder Alkoxy mit 1—4 Kohlenstoffatomen monosubstituiert ist, oder Thienyl oder eine gegebenenfalls substituierte Phenylgruppe a bedeutet,

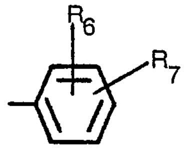

a

worin

$R_6$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet,

$R_7$ Wasserstoff, Alkyl mit 1—4 Kohlenstoffatomen, Alkoxy mit 1—4 Kohlenstoffatomen oder Halogen bedeutet, oder

$R_6$ und $R_7$ an benachbarte Kohlenstoffatome gebunden sind und zusammen Methylendioxy oder Äthylendioxy bedeuten,

sowie deren Säureadditionssalze.

Sofern in den Verbindungen der Formel I die Substituenten $R_1$, bis $R_4$ der Phenylringe und die in dem Rest $R_5$ enthaltenen Substituenten eine niedere Alkylgruppe enthalten, kann diese gerade oder verzweigt sein und 1—4 Kohlenstoffatome enthalten. So kommen insbesondere Methyl, Äthyl, n-Propyl, Isopropyl, n-Butyl oder tert.-Butyl, bevorzugt Methyl, Äthyl, n-Propyl und Isopropyl, in Frage. Insbesondere bei Disubstitution an den Phenylringen sind als Alkyl Äthyl und insbesondere Methyl bevorzugt. Niedermolekulare Alkoxysubstituenten stellen vorzugsweise Methoxy oder Äthoxy dar.

Als Halogensubstituenten in den Phenylringen und/oder einem Pyridylring kommen insbesondere Fluor, Chlor oder Brom in Frage. Falls ein Phenylring durch Trifluormethyl substituiert ist, ist Monosubstitution bevorzugt. Im Falle von Halogen und/oder Alkyl bzw. und/oder Alkoxy Substituenten ist Mono- oder Disubstitution zweckmäßig.

Die Gruppe Z stellt eine gerade oder verzweigte Alkylenkette mit 2—6 Kohlenstoffatomen dar, wobei Alkylenketten mit 2—4 Kohlenstoffatomen bevorzugt sind.

Falls $R_5$ für eine Pyridyl-Gruppe steht, kann diese in 2-, 3- oder 4-Stellung, insbesondere 2-Stellung, mit dem Restmolekül verbunden sein. Die Pyridyl-Gruppe kann unsubstituiert oder durch einen der vorgehend genannten Substituenten, insbesondere niederes Alkyl oder Alkoxy, vorzugsweise Methyl oder Methoxy substituiert sein.

Falls $R_5$ für eine Thienyl-Gruppe steht, kann diese in 2- oder 3-Stellung, vorzugsweise in 2-Stellung, mit dem Restmolekül verbunden sein.

Erfindungsgemäß werden die neuen 1,5-Diphenylpyrazolin-3-on-Verbindungen der Formel I und deren Säureadditionssalze erhalten, indem man in an sich bekannter Weise

a) Verbindungen der Formel II oder III

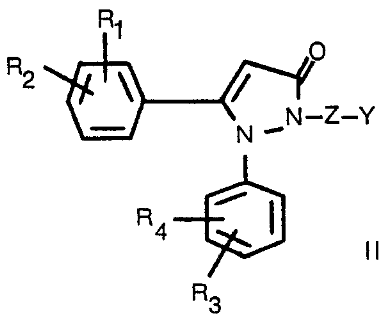

II

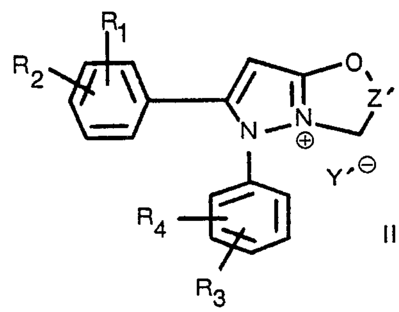

III

worin $R_1$, $R_2$, $R_3$ und $R_4$ obige Bedeutung besitzen und Z obige Bedeutung besitzt und Y einen aminolytisch abspaltbaren Rest bedeutet oder Z′ eine Alkylenkette mit 2—4 Kohlenstoffatomen und Y′ Halogen bedeuten, oder deren Gemische mit einer Verbindung der Formel V

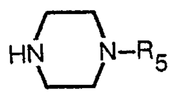

V

worin $R_5$ obige Bedeutung besitzt, umsetzt, oder

b) zur Herstellung von Verbindungen der Formel Ia

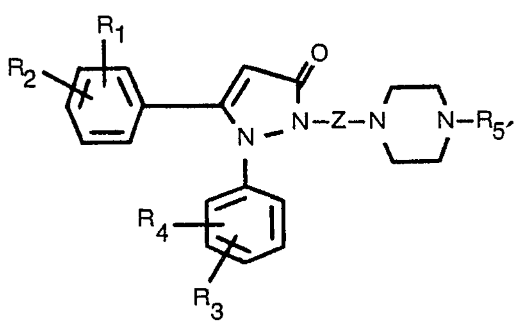

Ia

worin $R_1$, $R_2$, $R_3$, $R_4$ und Z obige Bedeutung besitzen und
$R_5'$ für eine substituierte Phenylgruppe a′ steht

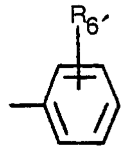

a′

worin $R_6'$ ortho- oder paraständig ist und $CF_3$ bedeutet, Verbindungen der Formel IV

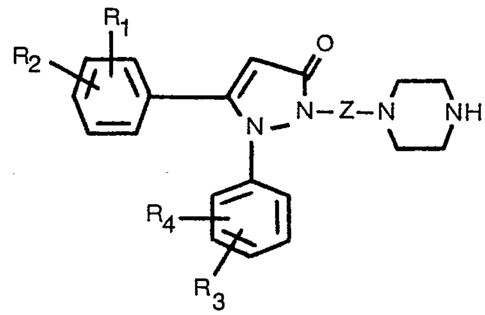

IV

worin $R_1$, $R_2$, $R_3$, $R_4$ und Z obige Bedeutung besitzen, mit Verbindungen der Formel VI

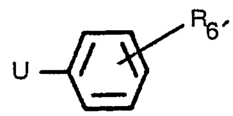

VI

worin $R_6'$ obige Bedeutung besitzt und U Halogen bedeutet, umsetzt,
und gegebenenfalls freie Verbindungen der Formel I in ihre Säureadditionssalze überführt oder die Säureadditionssalze in die freien Verbindungen der Formel I überführt.

Die Umsetzung von Verbindungen der Formel II oder III oder deren Gemischen mit Verbindungen der Formel V gemäß Verfahrensvariante a) kann nach an sich zur Alkylierung von Aminen üblichen Methoden ausgeführt werden.

Die Umsetzung von zweckmäßigerweise in einem unter den Reaktionsbedingungen inerten organischen Lösungsmittel unter basischen Bedingungen durchgeführt. Als aminolytisch abspaltbare Reste in Verbindungen der Formel II kommen insbesondere Halogene wie Chlor, Brom oder Jod, vorzugsweise Chlor oder Brom, in Frage. Als Beispiele geeigneter Lösungsmittel seien genannt aromatische Kohlenwasserstoffe wie Benzol, Toluol oder Xylol, cyclische Äther wie Dioxan, Dimethylformamid, 1,3-Dimethyl-2-imidazolidinon, Hexamethylphosphortriamid, Sulfolan, Dimethylsulfoxid, Tetramethylharnstoff oder niedere Alkanole, beispielsweise Isopentanol. Die Temperatur kann zwischen Raumtemperatur und 150°C betragen, wobei bei Verwendung von Verbindungen der Formel II zweckmäßig erhöhte Temperaturen, z. B. Temperaturen zwischen 50 und 150°C, insbesondere 90 und 150°C, verwendet werden, während bei Verwendung von Verbindungen der Formel III Temperaturen zwischen Raumtemperatur und Siedetemperatur des Lösungsmittels verwendet werden können. Gewünschtenfalls kann die Umsetzung der Verbindungen der Formel II oder III mit Verbindungen der Formel V jedoch auch ohne Lösungsmittel in der Schmelze stattfinden. Zweckmäßig kann man die Reaktion unter Zusatz einer organischen oder anorganischen Base durchführen. Man kann jedoch auch einen Überschuß der Verbindung der Formel V verwenden und diesen als interne Base benutzen. Geeignete anorganische Basen sind insbesondere Alkalimetallkarbonate oder -bikarbonate wie Natriumkarbonat, Natriumbikarbonat oder Kaliumkarbonat. Als organische Basen sind tertiäre organische Amine geeignet, insbesondere tertiäre Niederalkylamine wie Triäthylamin, n-Tripropylamin, n-Tributylamin, 1,4-Dimethylpiperazin oder Pyridin.

Falls die Verbindungen der Formel II, III oder V als Substituenten freie Hydroxy-Gruppen enthalten, werden diese zweickmäßigerweise während der Umsetzung in an sich bekannter Weise mit einer Schutzgruppe versehen. Geeignete nach der Reaktion leicht wieder abspaltbare Schutzgruppen sind zum Beispeil bekannt aus E. McOmie "Protective Groups in Organic Chemistry" Plenum Press 1971. Beispielsweise eignen sich zum Schutze einer Hydroxylfunktion Äther, insbesondere Tetrahydropyranyläther. Diese Schutzgruppen können nach der Umsetzung in bekannter Weise leicht wieder entfernt werden.

Die Umsetzung von Verbindungen der Formel IV mit Verbindungen der Formel VI kann ebenfalls auf an sich bekannte Weise unter zur Alkylierung von Aminen üblichen Bedingungen, beispielswiese den vorstehend für die Umsetzung von Verbindungen der Formel II mit Verbindungen der Formel V genannten Bedingungen, erfolgen. Die substituierten halogenierten Phenylverbindungen sind durch die Anwesenheit eines Substituenten zweiter Ordnung genügend aktiviert, um zur Umsetzung mit dem Piperazinderivat der Formel IV befähigt zu sein.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit Chlorwasserstoffsäure, Bromwasserstoffsäure, Schwefelsäure, Phosphorsäure, Methansulfonsäure, Äthansulfonsäure, Benzolsulfonsäure, p-Toluolsulfonsäure, Zitronensäure,

Essigsäure, Milchsäure, Bernsteinsäure, Maleinsäure, Fumarsäure, Äpfelsäure, Weinsäure, Benzoesäure, Phenylessigsäure oder Mandelsäure.

Die Verbindungen der Formel I enthalten 2 oder, falls $R_5$ eine gegebenenfalls substituierte Pyridylgruppe bedeutet, auch 3 basische Zentren und können somit Säureadditionssalze mit 1, 2 oder auch 3 Äquivalenten Säure bilden. Zur Herstellung von pharmazeutischen Zubereitungen eignen sich insbesondere monosaure Salze. Salze, welche mehrere Äquivalente Säure enthalten, können gewünschtenfalls in an sich bekannter Weise in monosaure Salze überführt werden, z. B. durch überführen in die freie Base und anschließende Umsetzung der Base mit einer äquivalenten Menge Säure.

Verbindungen der Formel I, worin Z eine verzweigte Alkylenkette darstellt, werden bei der Synthese in Form ihrer Racemate erhalten. Unter den Schutz dieser Erfindung fallen die racemischen Gemische wie auch die optisch aktiven Formen dieser Verbindungen. Die optisch aktiven Verbindungen können aus den racemischen Gemischen in an sich bekannter Weise durch Umsetzung mit geeigneten optisch aktiven Säuren, wie beispielsweise Weinsäure, O,O'-Dibenzoyl-Weinsäure, Mandelsäure, Di-O-isopropyliden-2-oxo-L-gulonsäure, und anschließend fraktionierte Kristallisation der gewonnenen Salze in ihre optisch aktiven Antipoden aufgetrennt werden (Tetrahedron *33*, (1977) 2725—2736).

Verbindungen der Formel II und III sind in der Literatur bisher noch nicht beschreiben worden und stellen neue, wertvolle Zwischenprodukte für die Herstellung von pharmakologisch aktiven Verbindungen, beispielsweise den Verbindungen der Formel I dar.

Verbindungen der Formel II und III können nach an sich bekannten Verfahren erhalten werden. So kann man insbesondere Verbindungen der Formel II, worin Y Halogen bedeutet, und Verbindungen der Formel III erhalten, indem man in situ hergestellte Alkalimetallsalze von 1,5-Diphenylpyrazolin-3-on-Verbindungen der Formel VIII

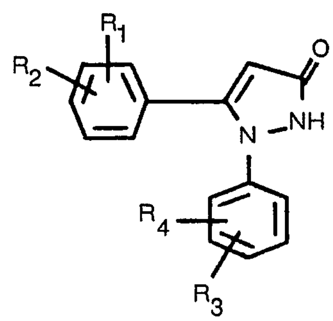

VIII

worin $R_1$, $R_2$, $R_3$ und $R_4$ obige Bedeutung besitzen, mit Verbindungen der Formel VII,

$$Y—Z—Y' \qquad VII$$

worin Z und Y obige Bedeutung besitzen und Y' Halogen bedeutet, umsetzt. Y' stellt vorzugsweise Chlor oder Brom dar.

Die Umsetzung erfolgt zweckmäßigerweise in einem unter den Reaktionsbedingungen inerten Lösungsmittel bei Temperaturen zwischen 0°C und Siedetemperatur des Lösungsmittels. Im allgemeinen sind Temperaturen zwischen 0°C und 100°C vorteilhaft. Als Lösungsmittel eignen sich beispielsweise niedere Alkohole wie Methanol, Äthanol, Isopropanol, Butanol oder tert.-Butanol, aber auch aromatische Kohlenwasserstoffe wie Benzol oder Toluol, Dimethylformamid, Sulfolan, Hexamethylphosphortriamid, Tetramethylharnstoff oder cyclische Äther, wie z. B. Dioxan oder Tetrahydrofuran.

Als Alkalimetallsalze der 1,5-Diphenylpyrazolin-3-on-Verbindungen kommen Lithium-, Natrium- oder Kaliumsalze, vorzugsweise das Natriumsalz, in Frage, welche in situ durch Umsetzen der Verbindungen der Formel VIII mit Alkalimetallalkoholaten oder Alkalimetallhydriden erhalten werden.

Sofern Z in den Verbindungen der Formel VII eine Alkylenkette mit 2—4 Kohlenstoffatomen darstellt, werden bei der Umsetzung neben den offenen Alkylierungsprodukten der Formel II auch cyclische Alkylierungsprodukte der Formel III erhalten. Die Verbindungen II und III liegen in dem Reaktionsgemisch in wechselnden Mengenverhältnissen vor in Abhängigkeit von den verwendeten Lösungsmitteln und Alkalimetallverbindungen, der Reaktionszeit und der jeweiligen Bedeutung der einzelnen Substituenten. So bilden sich z. B. bei Verwendung eines niederen Alkohols und des entsprechenden Alkalimetallalkoholats und längeren Reaktionszeiten, z. B. 12—35 Stunden, überwiegend die cyclischen Verbindungen III, während bei Verwendung von Dimethylformamid und Alkalimetallhydriden und Reaktionszeiten von z. B. 1—5 Stunden überwiegend die Verbindungen II entstehen. Da beide Verbindungen II und III oder deren Gemische in der folgenden Reaktion eingesetzt werden können, ist eine Trennung der beiden Verbindungen von der weiteren Umsetzung nicht nötig. Selbstverständlich aber können die cyclischen Verbindungen III durch Kristallisation von den offenkettigen Produkten in an sich bekannter Weise getrennt werden. So lassen sich die cyclischen Immoniumsalze III z. B. aus aromatischen und/oder halogenierten Kohlenwasserstoffen wie Benzol, Toluol oder Chloroform, leicht auskristallisieren.

Bei der Alkylierung der 1,5-Diphenylpyrazolin-3-on-Verbindungen der Formel VIII mit den Verbindungen der Formel VII werden im allgemeinen Gemische aus dem gewünschten N-alkylierten Produkt und

dem dazu isomeren O-alkylierten Produkt erhalten. Aus diesen Gemischen kann das N-alkylierte Produkt chromatographisch oder durch Kristallisation abgetrennt werden.

Die O-alkylierten Nebenprodukte können durch einfaches Erhitzen in die entsprechenden N-alkylierten Produkte II und die cyclischen Immoniumsalze III umgelagert werden. Die Umlagerungstemperatur liegt zweckmäßigerweise zwischen 60°C und 200°C. Gewünschtenfalls kann die Umlagerung in Gegenwart eines inerten Lösungsmittels zweckmäßigerweise bei Siedetemperatur des Lösungsmittels durchgeführt werden. Geeignete Lösungsmittel sind in dem angegebenen Bereich siedende niedere Alkohole, beispielsweise Methanol, Butanol oder Isopentanol, oder aromatische Kohlenwasserstoffe wie Benzol, Toluol oder Xylol. Für die thermische Umlagerungsreaktion können auch die bei der Alkylierung anfallenden Gemische aus cyclischen Immoniumverbindungen III, N-alkyliertem Produkt II und dem dazu isomeren O-alkylierten Produkt direkt ohne vorherige Trennung eingesetzt werden.

Verbindungen der Formel IV sind in der Literatur bisher noch nicht beschrieben worden und stellen neue wertvolle Zwischenprodukte für die Herstellung von pharmakologisch aktiven Verbindungen, beispielsweise den Verbindungen der Formel I, dar.

Verbindungen der Formel IV können nach an sich bekannten Methoden erhalten werden, indem man beispielsweise Verbindungen der Formel II oder III mit einem Überschuß Piperazin umsetzt. Die Umsetzung kann nach an sich zur Alkylierung von Aminen üblichen Methoden, beispielsweise unter den vorstehend für die Umsetzung von Verbindungen der Formel II oder III mit Verbindungen der Formel V beschriebenen Bedingungen, durchgeführt werden.

Verbindungen der Formel IV können auch aus Verbindungen der Formel IX

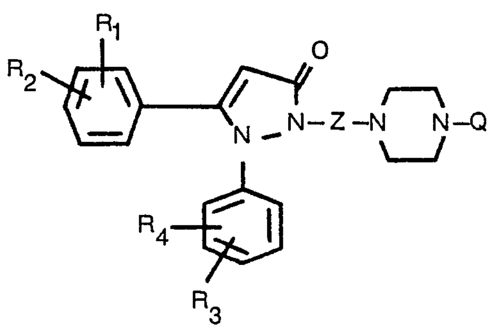

IX

worin $R_1$, $R_2$, $R_3$, $R_4$ und Z obige Bedeutung besitzen und Q für eine Aminschutzgruppe steht, erhalten werden, indem man die Aminschutzgruppe auf an sich bekannte Weise abspaltet. Als Aminschutzgruppen kommen die an sich bekannten zum Schutz einer Aminofunktion gebräuchlichen Schutzgruppen, beispielsweise hydrolytisch abspaltbare Acylgruppen oder hydrogenolytisch abspaltbare Benzylgruppen in Frage. Geeignete Schutzgruppen sind z. B. bekannt aus E. McOmie "Protective Groups in Organic Chemistry"; Plenum Press, London (1971), S. 44ff. Insbesondere eignen sich die Formylgruppe und niedere Carbalkoxyschutzgruppen. Diese können in an sich bekannter Weise durch saure oder alkalische Hydrolyse abgespalten werden.

Verbindungen der Formel IX können auf an sich bekannte Weise erhalten werden, beispielsweise durch Umsetzung von Verbindungen der Formel II oder III mit Verbindungen der Formel X

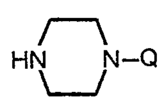

X

worin Q obige Bedeutung besitzt. Die Umsetzung kann nach zur Alkylierung von Aminen üblichen Methoden, beispielsweise unter den für die Umsetzung von Verbindungen der Formel II oder III mit Verbindungen der Formel V beschriebenen Reaktionsbedingungen, erfolgen.

Die 1,5-Diphenyl-pyrazolin-3-one der Formel VIII sind bekannt oder können nach an sich bekannten Methoden, beispielsweise den von Michaelis und Rassmann (Ann. *352*, (1907) 158) und den von Michaelis und Willert (Ann. *358*, (1908) 159) beschriebenen Methoden hergestellt werden, z. B. ausgehend von entsprechend substituierten Benzoylessigsäureestern und entsprechend substituierten β-Acetyl-phenylhydrazinen.

Verbindungen der Formel V sind bekannt oder können nach an sich bekannten Methoden hergestellt werden, beispielsweise durch Umsetzung von Aminen der Formel XI

$$H_2N—R_5 \qquad XI$$

worin $R_5$ obige Bedeutung besitzt, mit entsprechenden Di(halogenalkyl)aminen unter zur Alkylierung von Aminen üblichen Bedingungen.

Die Verbindungen der Formel I und ihre pharmakologisch akzeptablen Säureadditionssalze zeichnen sich durch interessante pharmakologische Eigenschaften aus und zeigen insbesondere antiallergische Wirkungen. Außerdem besitzen die Verbindungen eine gute Verträglichkeit bei nur geringer Toxizität und zeigen insbesondere einen größen Abstand zwischen therapeutisch wirksamer und toxischer Dosis.

Aufgrund ihrer antiallergischen Wirkungen eignen sich die Verbindungen der Formel I und ihre pharmakologisch akzeptablen Salze als Antiallergika zur Behandlung allergischer Erkrankungen wie z. B. Bronchialasthma oder allergischer Rhinitis.

Die antiallergischen Eigenschaften der Verbindungen der Formel I können in pharmakologischen Standardtests an kleinen Tieren nachgewiesen werden. Beispielsweise weisen die Substanzen inhibierende Wirkungen gegenüber der zu allergischen Reaktionen führenden Freisetzung endogener Mediatoren aus den Mastzellen oder basophilen Leukozyten auf. Die zu verwendenden Dosen varieeren naturgemäß je nach Art der verwendeten Substanz, der Applikationsart und des zu behandelnden Zustandes. Im allgemeinen werden jedoch befriedigende Ergebnisse in Tierversuchen mit Dosen zwischen 0,05 und 75 mg pro kg Körpergewicht erhalten. So zeigen die neuen Verbindungen eine spezifische hemmende Wirkung in dem nachstehend beschriebenen PCA-Test (Passive Cutane Anaphylaxie) an der Ratte.

Beschreibung der Testmethode zur Bestimmung der Hemmung der passiven cutanen Anaphylaxie (PCA-Test siehe Arch.int. pharmacology *252* (1981) 316—326).

Zur Herstellung des in dem Test verwendeten IgE-Antiovoalbuminserums nach der Methode von Mota (Immunology *7*, (1964) 681) und J. Goose (Immunology *16* (1969) 749) wurden männliche Wister-Ratten von 200—250 g Körpergewicht durch s.c.-Injektion von 1 mg Ovoalbumin und 1 ml Bordetella-Pertussis-Suspension (Vaxicoq® Merieux $3 \cdot 10^{10}$ Organismen/ml) sensibilisiert. Nach 14 Tagen werden die Tiere entblutet und das Blut zentrifugiert. Das so erhaltene Antiserum wird bei 20°C gelagert.

An je vier verschiedenen Stellen des rasierten Rückens von nicht sensibilisierten Ratten werden je 0,1 ml Antiserum in die Haut injiziert. Nach 72 Stunden werden eine Lösung der Testverbindung oder zum Vergleich nur das Lösungsmittel oral appliziert und 10 Minuten später 5 mg Ovoalbumin und 5 mg blauer Farbstoff (Evans Blue) in 0,9 %iger NaCl-Lösung i.p. appliziert. Nach 30 Minuten werden die Tiere getötet und die Durchmesser der an den mit Antiserum injizierten Stellen entstandenen blauen Flecken festgestellt. Der Hemmeffekt der Testsubstanz wird aus der Größe der auftretenden blauen Flecken bestimmt.

Die folgende Tabelle gibt nach dem vorstehend beschriebenen Test erhaltene Ergebnisse wieder. Die für die Verbindungen der Formel I angegebenen Beispielsnummern beziehen sich auf die nachstehenden Herstellungsbeispiele.

| Test-Substanz der Formel I Beispiel Nr. | P.C.A. Hemmung $EO_{50}$ mg/kg p.o. |
|---|---|
| 1 | 0,8 |
| 13 | 8,5 |
| 22 | 9,8 |

Bestimmung der minimalen toxischen Dosis an der Maus. Bei oraler Verabreichung der vorstehenden Substanzen in Dosen von bis zu 300 mg/kg konnten keine toxischen Symptome festgestellt werden.

Als Heilmittel können die Verbindungen der Formel I und ihre pharmakologisch verträglichen Salze zusammen mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z. B. Talkum, Milchzucker oder Stärke oder flüssiger Verdünnungsmittel wie z. B. Wasser, fetten Ölen oder flüssigen Paraffinen.

Die Verbindungen der Formel I können in pharmazeutischen Gebrauchsformen verabreicht werden, welche etwa 0,5 bis 100 mg, vorzugsweise 0,5—25 mg Aktivsubstanz pro Einzeldosis enthalten. Die zu verwendende Dosierung wird selbstverständlich der zu behandelnden Spezies und den individuellen Erfordernissen angepaßt werden. Parenterale Formulierungen werden im allgemeinen weniger Aktivsubstanz enthalten als Präparate zur oralen Verabreichung.

Die nachfolgenden Beispiele sollen die Herstellung der neuen Verbindungen der Formel I sowie der neuen Zwischenprodukte näher erläutern jedoch den Umfang der Erfindung in keiner Weise beschränken.

Die Strukturen der neuen Verbindungen wurden durch spektroskopische Untersuchungen, insbesondere durch eine genaue Analyses der IR- und NMR-Spektren gesichert.

Die IR-Spektren der 1,5-Diphenylpyrazolin-3-on-Verbindungen zeigen bei ca. 1630—1680 $cm^{-1}$ die Carbonyl-Absorptionsbande des Pyrazolin-3-on-ringes und sind brei von —C=N-Banden, die in Pyrazolderivaten beobachtet werden können.

Beispiel 1

1,5-Diphenyl-2-{3-[4-(2-pyridyl)-piperazin-1-yl]-propyl}-pyrazolin-3-on.

A) 47,2 g 1,5-Diphenylpyrazolin-3-on (200 mMol) werden in 350 ml Dimethylformamid gelöst. Der Lösung werden unter Rühren bei 80°C portionsweise 6,6 g Natriumhydrid (80 %ig, 220 mMol) zugesetzt.

Man läßt die erhaltene Suspension auf 60°C abkühlen und tropft eine Lösung von 34,7 g (220 mMol) 1-Brom-3-chlorpropan in 150 ml Dimethylformamid hinzu. Die Temperatur sinkt hierbei auf ca. 40°C ab. Bei dieser Temperatur wird 12 Stunden weitergerührt. Anschließend wird das Dimethylformamid weitestgehend unter vermindertem Druck (Ölpumpe) abgezogen, wobei auch unumgesetztes Bromchlorpropan aus der Reaktionsmischung entfernt wird. Der Rückstand wird in Methylenchlorid aufgenommen und gerührt. Hierbei fallen Natriumbromid und unumgesetztes Diphenylpyrazolinon aus und können abgesaugt werden. Die Methyenchloridlösung wird mit Wasser gewaschen, über Natriumsulfat getrocknet, filtriert und das Lösungsmittel unter vermindertem Druck abgezogen.

Es verbleiben 60 g eines zähen, hellgelben Öles, welches ein Gemisch der Isomeren 1,5-Diphenyl-2-(3-chlorpropyl)pyrazolin-3-on und 1,5-Diphenyl-3-(3-chlorpropoxy)-pyrazol enthält. Letzteres wird durch einstündiges Erhitzen des Gemisches auf 170°C ebenfalls in das 1,5-Diphenyl-2-(3-chlorpropyl)-pyrazolin-3-on umgelagert. Das erhaltene Produkt kann ohne weitere Reinigung in die nächste Reaktionsstufe eingesetzt werden.

B) 31,3 g 1,5-Diphenyl-2-(3-chlorpropyl)-pyrazolin-3-on werden in 300 ml Toluol gelöst und die Lösung mit 16,3 g N-(2-Pyridyl)-piperazin und 15,9 g Kaliumcarbonat versetzt. Unter Rühren wird die Suspension 20 Stunden unter Rückfluß erhitzt. Nach dem Erkalten wird das Reaktionsgemisch mit Wasser ausgeschüttelt, die organische Phase weitestgehend unter vermindertem Druck eingeengt, der

Rückstand mit verdünnter Salzsäure aufgenommen und die so erhaltene Suspension mit Methylenchlorid extrahiert. Die nunmehr klare wäßrige Phase wird mit verdünnter Natronlauge alkalisch gestellt und mit Äthylacetat extrahiert. Der organische Extrakt wird über Natriumsulfat getrocknet, filtriert und unter vermindertem Druck eingedampft. Als Rückstand verbleiben 42,4 g der Titelverbindung als hellgelbes Öl.

Dieser Rückstand wird zur Bildung des Trihydrochlorids der Titelverbindung in Isopropylalkohol gelöst und die Lösung mit einer gesättigten Lösung von Chlorwasserstoff in Diäthyläther tropfenweise unter Rühren versetzt. Die ausgefallenen Trihydrochlorid-Kristalle werden abgesaugt und mit Isopropylalkohol und Äther nachgewaschen. Ausbeute: 41,6 g. Schmelzpunkt 196—198°C.

Beispiel 2

1,5-Diphenyl-2-{3-[4-(2-pyridyl)-piperazin-1-yl]-propyl}-pyrazolin-3-on.

A) 118 g 1,5-Diphenylpyrazolin-3-on werden in 1 l Methanol suspendiert und unter Rühren eine Lösung von 99 g Natriummethylat in Methanol (30%ige Lösung) zugegeben. Zu der entstandenen klaren Lösung werden nach 15 Minuten tropfenweise 54 ml 1-Brom-3-chlorpropan gegeben und die Lösung 48 Stunden unter Rückfluß erhitzt. Dann wird die Hauptmenge des Lösungsmittels unter vermindertem Druck abdestilliert, der Rückstand in 1 l Dichlormethan gelöst und die organische Lösung zweimal mit je 250 ml Wasser gewaschen. Das Waschwasser wird noch einmal mit 300 ml Methylenchlorid extrahiert und die organischen Phasen werden vereinigt, über Natriumsulfat getrocknet und filtriert. Nach Abziehen des Lösungsmittels unter vermindertem Druck verbleiben 150 g rohes 1,5-Diphenylpyrazolo[2,3-b]-dihydro-1,3-oxazinium-chlorid als gelblich kristalliner Ruckstand. Dieser Rückstand wird in 200 ml Aceton suspendiert, die Suspension 5 Minuten unter Rückfluß erhitzt und dann unter Rühren gekühlt. Die Kristalle werden abfiltriert und nochmals in gleicher Weise mit 250 ml Äthylacetat gewaschen. Anschließend werden die Kristalle in einer Trockenkammer bei 60°C 5 Stunden getrocknet. Schmelzpunkt 208—210°C, Ausbeute 102 g.

B) 156,3 g 1,5-Diphenylpyrazolo-[2,3-b]-dihydro-1,3-oxazinium-Chlorid werden in 1,5 l Toluol suspendiert, 165 g Kaliumkarbonat zugegeben und die Suspension auf 50°C erhitzt. Unter Rühren werden innerhalb 10 Minuten 77 ml 1-(2-Pyridyl)-piperazin zugetropft und die Reaktionsmischung sodann 5 Stunden am Rückflüß erhitzt. Nach Abkühlen wird mit 650 ml 15%iger wäßriger Salzsäure angesäuert, die wässrige Phase von der organischen Phase abgetrennt und mit 300 ml Äthylacetat gewaschen. Die organischen Phasen werden verworfen. Zu der wäßrigen Phase wird eine Lösung von 175 g Natriumhydroxid in 175 ml Wasser gegeben und die Mischung wird dreimal mit je 250 ml Methylenchlorid extrahiert. Die vereinigten Methylenchloridphasen werden über Natriumsulfat getrocknet und filtriert. Nach Entfernen des Lösungsmittels unter vermindertem Druck verbleiben 225 g rohes 1,5-Diphenyl-2-{3-[4-(2-pyridyl)-piperazin-1-yl]-propyl}-pyrazolin-3-on als gelbes Öl zurück. Dieses wird in 4,5 l Isopropanol gelöst. Dann wird unter Rühren ein Überschuß an Chlorwasserstoffgas eingeleitet und 1 Stunde gerührt. Der gebildete kristalline Niederschlag wird abfiltriert, zweimal mit je 250 ml Isopropanol gewaschen und in einer Trockenkammer bei 60°C 12 Stunden getrocknet. Es werden 212 g des Trihydrochlorids der Titelverbindung mit Schmelzpunkt 196—198°C erhalten.

C) Überführung des Trihydrochlorides der Titelverbindung in das Monohydrochlorid: 212 g des Trihydrochlorides werden in 500 ml Wasser gelöst, eine Lösung von 55 g Natriumhydroxyd in 55 ml Wasser zugegeben und dreimal mit je 250 ml Methylenchlorid extrahiert. Die organische Phase wird über Natriumsulfat getrocknet und filtriert. Nach Entfernen des Lösungsmittels unter vermindertem Druck bleibt die Base als farbloser öliger Rückstand. Dieser wird in 2,5 ml Isopropanol gelöst. Die Lösung wird unter Rühren mit einer zuvor hergestellten Lösung von 9 g gasförmigem Chlorwasserstoff in 500 ml Isopropanol zusammengegeben. Die hierbei sich bildende Niederschlag besteht aus dem Monohydrochlorid der Titelverbindung. Es wird noch 1 Stunde lang gerührt und dann werden die Kristalle abfiltriert, dreimal mit je 200 Isopropanol gewaschen und in einer Trockenkammer bei 60°C 12 Stunden lang getrocknet. Schmelzpunkt 206—208°C, Ausbeute 164 g.

Beispiel 3

1,5-Diphenyl-2-[3-(4-phenylpiperazin-1-yl)-propyl]-pyrazolin-3-on.

31,3 g 1,5-Diphenyl-2-(3-chlorpropyl)-pyrazolin-3-on (hergestellt analog Beispiel 1A) werden in 300 ml Toluol gelöst und die Lösung mit 18,6 g N-Phenylpiperazin und 15,9 g Kaliumcarbonat versetzt. Unter Rühren wird die entstandene Suspension 20 Stunden unter Rückfluß erhitzt. Nach Erkalten wird die Reaktionslösung mit Wasser ausgeschüttelt, die organische Phase abgetrennt und unter vermindertem Druck weitestgehend eingeengt. Der Rückstand wird mit verdünnter Salzsäure versetzt und die dabei erhaltene Suspension mit Methylenchlorid extrahiert. Die nunmehr klare wäßrige Phase wird mit verdünnter Natronlauge bis zur alkalischen Reaktion versetzt und mit Äthylacetat extrahiert. Der organische Extrakt wird abgetrennt, über Natriumsulfat getrocknet, filtriert und unter vermindertem Druck eingedampft. Das rohe 1,5-Diphenyl-2-[3-(4-phenylpiperazin-1-yl]-propyl)-pyrazolin-3-on bleibt als hellgelbes Öl zurück (41,2 g).

Dieser Rückstand wird zur Bildung des Dihydrochlorides in Isopropanol gelöst und die Lösung mit einer gesättigten Lösung von Chlorwasserstoff-Gas in Diäthyläther tropfenweise unter Rühren versetzt. Das kristallin ausgefallene Dihydrochlorid wird abgesaugt und mit Isopropanol und Äther nachgewaschen. Schmelzpunkt 227—230°C, Ausbeute 38,7 g.

Beispiel 4

1,5-Diphenyl-2-{3-[4-(4-trifluormethylphenyl)-piperazin-1-yl]-propyl}-pyrazolin-3-on.

A) 24,2 g des nach Beispiel 2A hergestellten 1,5-Diphenylpyrazolo-[2,3-*b*]-dihydro-oxazinium-chlorid werden in 200 ml Isopropanol mit 22,8 g N-Formylpiperazin und 2,5 g Kaliumbromid 12 Stunden unter Rückfluß erhitzt. Danach wird das Isopropanol im Vakuum abdestilliert und der Rückstand in Toluol aufgenommen. Die Toluolphase wird mit verdünnter Salzsäure extrahiert, die salzsauren Extrakte mit verdünnter Natronlauge bis zur alkalischen Reaktion versetzt und mit Methylenchlorid extrahiert. Die Methylenchloridphase wird abgetrennt, neutral gewaschen, über Natriumsulfat getrocknet und in Vakuum eingedampft. Als Rückstand erhält man 1,5-Di-phenyl-2-[3-(4-formylpiperazin-1-yl)-propyl]-pyrazolin-3-on.

B) 19,2 g 1,5-Diphenyl-2-[3-(4-formylpiperazin-1-yl]-propyl)-pyrazolin-3-on werden in 200 ml eines Gemisches Äthanol und 20 %igere Salzsäure (1:1) gelöst. Die Lösung wird 12 Stunden bei Raumtemperatur stehengelassen, anschließend noch 2 Stunden am Rückfluß erhitzt und dann das Äthanol im Vakuum abdestilliert. Der Rückstand wird mit Toluol und verdünnter Natronläuge versetzt. Die Toluolphase wird abgetrennt, mit Wasser gewaschen, über Natriumsulfat getrocknet und im Vakuum eingedampft. Als Rückstand wird 1,5-Diphenyl-2-[3-(piperazin-1-yl)-propyl]-pyrazolin-3-on erhalten.

C) 13,3 g der vorstehend erhaltenen Piperazinverbindung werden in 150 ml Dimethylformamid gelöst und die Lösung mit 7 g Kaliumcarbonat und 8,5 g 4-Trifluormethyl-brombenzol versetzt und unter Rühren 16 Stunden auf 120°C erhitzt. Danach zieht man das Lösungsmittel weitgehend unter vermindertem Druck ab, nimmt den dunkelbraunen Rückstand mit verdünnter Salzsäure auf und extrahiert mit Äthylacetat, welches danach verworfen werden kann. Die wäßrige Phase wird danach wieder alkalisch gestellt und mit Dichlormethan extrahiert. Die organische Phase wird über Natriumsulfat getrocknet und über eine kurze Kieselgelsäule filtriert. Nach Eindampfen des Lösungsmittels verbleibt die Titelverbindung als farbloses, zähes Öl.

Nach den in Beispiel 1—4 beschriebenen Verfahren können auch die in der folgenden Tabelle aufgeführten 1,5-Diphenyl-2-[(piperazin-1-yl)-alkyl]-pyrazolin-3-on-Verbindungen der Formel I aus entsprechenden Verbindungen der Formel II, III oder IV hergestellt werden.

| Bsp.Nr. | $R_1$ | $R_2$ | $R_3$ | $R_4$ | Z | $R_5$ | Salz | Fp °C |
|---|---|---|---|---|---|---|---|---|
| 5 | H | H | H | H | $C_2H_4$ | 2-Cl-Phen | Di-HCl | 215 |
| 6 | H | H | H | H | n-$C_3H_6$ | 2-Cl-Phen | Di-HCl | 162-164 |
| 7 | H | H | H | H | n-$C_4H_8$ | 2-Cl-Phen | Di-HCl | 164-167 |
| 8 | H | H | H | H | n-$C_3H_6$ | 4-Cl-Phen | HCl-Hydrat | 238-240 |
| 9 | H | H | H | H | n-$C_3H_6$ | 2-$CH_3O$-Phen | Ma | 158-160 |
| 10 | H | H | H | H | $C_2H_4$ | 4-$CH_3O$-Phen | Base·0,5 n-$C_4H_9OCOCH_3$ | 105-107 |
| 11 | H | H | H | H | n-$C_3H_6$ | 4-$CH_3O$-Phen | Di-HCl | 201-203 |
| 12 | H | H | H | H | n-$C_4H_8$ | 4-$CH_3O$-Phen | Base | 74-76 |
| 13 | 4-Cl | H | H | H | n-$C_3H_6$ | 2-Pyr | HCl | 190-192 |
| 14 | H | H | H | H | n-$C_4H_8$ | 2-Pyr | HCl | 185-190 |
| 15 | H | H | H | H | $C_2H_4$ | 3,4-$OC_2H_4O$-Phen | Base | öl |
| 16 | H | H | 3,4di-$CH_3O$ | | n-$C_3H_6$ | Phen | Base | öl |
| 17 | 3-$CH_3COO$ | H | H | H | n-$C_3H_6$ | Phen | Base | öl |
| 18 | 3-OH | H | H | H | n-$C_3H_6$ | Phen | Base | öl |

| Bsp.Nr. | $R_1$ | $R_2$ | $R_3$ | $R_4$ | Z | $R_5$ | Salz | Fp °C |
|---|---|---|---|---|---|---|---|---|
| 19 | H | H | H | H | $n-C_4H_8$ | Phen | Base | öl |
| 20 | H | $2-CH_3$ | H | H | $C_2H_4$ | Phen | Base | öl |
| 21 | H | H | H | H | $n-C_4H_8$ | $2-CH_3O$-Phen | HCl | 151-152 |
| 22 | H | H | H | H | $n-C_3H_6$ | 4-F-Phen | Di-HCl | 185-190 (133-135 w) |
| 23 | H | H | H | H | $n-C_3H_6$ | $4-CH_3O$-Phen | Di-HCl | 220-222 |
| 24 | H | H | H | H | $n-C_3H_6$ | Phen | Base | öl |
| 25 | H | H | H | H | $n-C_4H_8$ | 3-Pyr | Base | öl |
| 26 | H | H | H | H | $C_2H_4$ | 4-Pyr | Base | öl |
| 27 | $3-CH_3$ | H | H | H | $C_2H_4$ | Phen | Base | öl |
| 28 | H | H | H | H | $n-C_4H_8$ | 2-Br-Phen | Base | öl |
| 29 | $3,4-OCH_2O$ | | 4-Cl | H | $n-C_3H_6$ | 2-F-Phen | Base | öl |
| 30 | H | H | $3-CH_3$ | H | $n-C_3H_6$ | Phen | Base | öl |

| Bsp.Nr. | $R_1$ | $R_2$ | $R_3$ | $R_4$ | Z | $R_5$ | Salz | Fp °C |
|---|---|---|---|---|---|---|---|---|
| 31 | H | H | H | H | $C_2H_4$ | 2-Pyr | Base | öl |
| 32 | H | H | H | H | n-$C_3H_6$ | 4-Pyr | HCl | 185-190 |
| 33 | H | H | H | H | n-$C_3H_6$ | 2-Br-Phen | Base | öl |
| 34 | H | H | H | H | n-$C_3H_6$ | 2-Thien | HCl | 170-175(Z) |
| 35 | H | H | H | H | n-$C_3H_6$ | 3-Thien | Base | öl |
| 36 | H | H | H | H | n-$C_3H_6$ | 4-OH-Phen | Base | öl |
| 37 | H | H | H | H | n-$C_3H_6$ | 3-$CF_3$-Phen | Di-HCl | 200-212 |
| 38 | H | H | H | H | n-$C_3H_6$ | 4-$CH_3COO$-Phen | Base | öl |
| 39 | 4-$CH_3$ | H | H | H | n-$C_3H_6$ | 2-Pyr | HCl | 180-182 |
| 40 | H | H | 2-Br | H | n-$C_3H_6$ | 2-Pyr | Base | 118-120 |
| 41 | H | H | 2-$CH_3$ | H | n-$C_3H_6$ | 2-Pyr | Base | 92-94 |
| 42 | H | H | 4-$CH_3O$ | H | n-$C_3H_6$ | 2-Pyr | Base | öl |
| 43 | H | H | 3-$CF_3$ | H | n-$C_3H_6$ | 2-Pyr | Base | öl |
| 44 | 4-$CH_3$ | H | 2-Br | H | n-$C_3H_6$ | 2-Pyr | Base | öl |
| 45 | 4-Cl | H | 4-$CH_3O$ | H | n-$C_3H_6$ | 2-Pyr | Base | öl |

| Bsp.Nr. | $R_1$ | $R_2$ | $R_3$ | $R_4$ | Z | $R_5$ | Salz | Fp °C |
|---|---|---|---|---|---|---|---|---|
| 46 | H | H | H | H | n-$C_3H_6$ | 2,6-di-$CH_3$-Phen | Base | 85-88 |
| 47 | H | H | H | H | n-$C_3H_6$ | 3,4-di-$CH_3O$-Phen | Base | 126-127 |
| 48 | H | H | H | H | n-$C_3H_6$ | 2-$CH_3O$-4-Cl-Phen | Base | öl |
| 49 | H | H | H | H | n-$C_3H_6$ | 2-$C_2H_5O$-Phen | Base | öl |
| 50 | H | H | H | H | n-$C_3H_6$ | 3-$CH_3$-Phen | Base | öl |
| 51 | H | H | H | H | n-$C_3H_6$ | 4-$CH_3$-Phen | Base | öl |
| 52 | H | H | 4-F | H | n-$C_3H_6$ | 2-Pyr | Base | öl |
| 53 | H | H | H | H | n-$C_3H_6$ | 3,4-O-$CH_2$-O-Phen | Base | öl |
| 54 | H | H | H | H | n-$C_4H_9$ | 4-F-Phen | Base | öl |
| 55 | 4-$CH_3$ | H | 2-Cl | H | n-$C_3H_6$ | 2-Pyr | Base | öl |
| 56 | 4-$CH_3O$ | H | H | H | n-$C_3H_6$ | 2-Pyr | Base | öl |

Phen = Phenyl
Pyr = Pyridyl
Thien = Thienyl

HCl = Hydrichlorid
Ma = Maleinat
Base = freie Base

öl = ölig
Z = Zersetzung
w = erweichen

Beispiel I
Tabletten

Man stellt Tabletten in folgender Zusammensetzung pro Tablette her:

| | |
|---|---|
| 1,5-Diphenyl-2-{3-[4-(2-pyridyl)-piperazin-1-yl]-propyl}-pyrazolin-3-on-monohydrochlorid | 25 mg |
| Maisstärke | 60 mg |
| Milchzucker | 130 mg |
| Gelatinelösung (10%ige Lösung) | 6 mg |

Die Wirkstoff, die Maisstärke und der Milchzucker werden mit der 10%igen Gelatine-Lösung eingedickt. Die Paste wird zerkleinert und das entstandene Granulat wird auf ein geeignetes Blech gebracht und bei 45°C getrocknet. Das getrocknete Granulat wird durch eine Zerkleinerungsmaschine geleitet und in einem Mixer mit weiteren folgende Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

**Patentansprüche für die Vertragsstaaten: BE CH DE FR GB IT LU NL SE**

1. 1,5-Diphenylpyrazolin-3-on-Verbindungen der allgemeinen Formel I

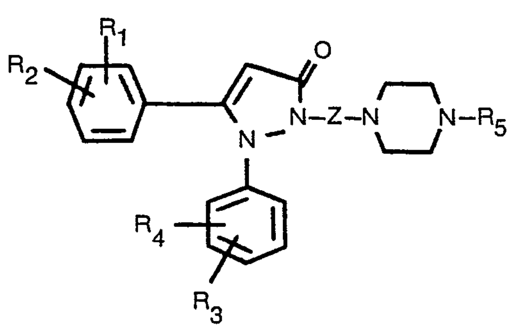

I

worin

$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_2$ Wasserstoff, Alkyl mit 1—4 Kohlenstoffatomen, Alkoxy mit 1—4 Kohlenstoffatomen oder Halogen bedeutet, oder

$R_1$ und $R_2$ an benachbarte Kohlenstoffatome gebunden sind und zusammen Methylendioxy oder Äthylendioxy bedeuten,

$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_4$ Wasserstoff, Alkyl mit 1—4 Kohlenstoffatomen, Alkoxy mit 1—4 Kohlenstoffatomen oder Halogen bedeutet, oder

$R_3$ und $R_4$ an benachbarte Kohlenstoffatome gebunden sind und zusammen Methylendioxy oder Äthylendioxy bedeuten,

Z eine Alkylengruppe mit 2—6 Kohlenstoffatomen bedeutet,

$R_5$ Pyridyl, welches unsubstituiert oder durch Alkyl mit 1—4 Kohlenstoffatomen, Halogen oder Alkoxy mit 1—4 Kohlenstoffatomen monosubstituiert ist, oder Thienyl oder eine gegebenenfalls substituierte Phenylgruppe a bedeutet

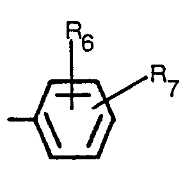

a

worin
$R_6$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und
$R_7$ Wasserstoff, Alkyl mit 1—4 Kohlenstoffatomen, Alkoxy mit 1—4 Kohlenstoffatomen oder Halogen bedeutet, oder
$R_6$ und $R_7$ an benachbarte Kohlenstoffatome gebunden sind und zusammen Methylendioxy oder Äthylendioxy bedeuten,
sowie deren Säureadditionssalze.

2. 1,5-Diphenylpyrazolin-3-on-Verbindungen gemäß Anspruch 1, worin Z, $R_1$, $R_2$, $R_3$ und $R_4$ obige Bedeutung besitzen und $R_5$ für eine gegebenenfalls substituierte Phenylgruppe a steht

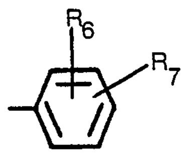
a

worin $R_6$ und $R_7$ obige Bedeutung besitzen.

3. 1,5-Diphenylazolin-3-on-Verbindungen gemäß Anspruch 1, worin Z, $R_1$, $R_2$, $R_3$ und $R_4$ obige Bedeutung besitzen und $R_5$ Pyridyl, welches unsubstituiert oder durch Alkyl mit 1—4 Kohlenstoffatomen, Halogen oder Alkoxy mit 1—4 Kohlenstoffatomen monosubstituiert ist, bedeutet.

4. 1,5-Diphenyl-2-{3-[4-(2-pyridyl)-piperazin-1-yl]-propyl}-pyrazolin-3-on und dessen Säureadditionssalze gemäß Anspruch 3.

5. Verbindungen der allgemeinen Formel II oder III

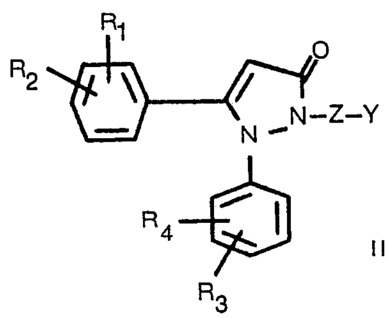
II

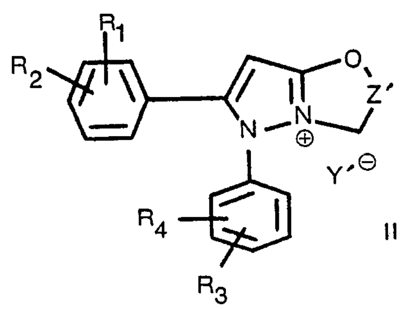
III

worin
$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und
$R_2$ Wasserstoff, Alkyl mit 1—4 Kohlenstoffatomen, Alkoxy mit 1—4 Kohlenstoffatomen oder Halogen bedeutet, oder
$R_1$ und $R_2$ an benachbarte Kohlenstoffatome gebunden sind und zusammen Methylendioxy oder Äthylendioxy bedeuten,
$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und
$R_4$ Wasserstoff, Alkyl mit 1—4 Kohlenstoffatomen, Alkoxy mit 1—4 Kohlenstoffatomen oder Halogen bedeutet,
$R_3$ und $R_4$ an benachbarte Kohlenstoffatome gebunden sind und zusammen Methylendioxy oder Äthylendioxy bedeuten, und
Z eine Alkylengruppe mit 2—6 Kohlenstoffatomen und
Y einen aminolytisch abspaltbaren Rest bedeuten, oder
Z' eine Alkylenkette mit 1—4 Kohlenstoffatomen und
Y' Halogen bedeuten,
und deren Gemische.

6. Verbindungen der Formel II gemäß Anspruch 5, worin $R_1$, $R_2$, $R_3$, $R_4$ und Z obige Bedeutung besitzen und Y Halogen bedeutet.

7. Verbindungen der allgemeinen Formel IV

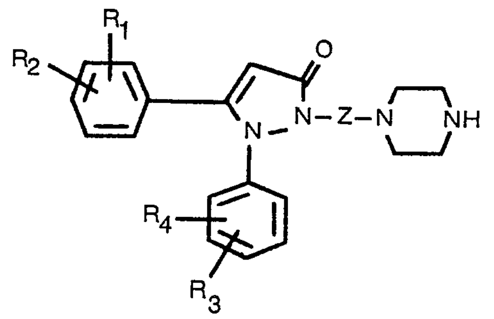

IV

worin

$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_2$ Wasserstoff, Alkyl mit 1—4 Kohlenstoffatomen, Alkoxy mit 1—4 Kohlenstoffatomen oder Halogen bedeutet, oder

$R_1$ und $R_2$ an benachbarte Kohlenstoffatome gebunden sind und zusammen Methylendioxy oder Äthylendioxy bedeuten,

$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_4$ Wasserstoff, Alkyl mit 1—4 Kohlenstoffatomen, Alkoxy mit 1—4 Kohlenstoffatomen oder Halogen bedeutet, oder

$R_3$ und $R_4$ an benachbarte Kohlenstoffatome gebunden sind und zusammen Methylendioxy oder Äthylendioxy bedeuten, und

Z eine Alkylengruppe mit 2—6 Kohlenstoffatomen bedeutet,

und deren Säureadditionssalze.

8. Verfahren zur Herstellung von 1,5-Diphenylpyrazolin-3-on-Verbindungen der allgemeinen Formel I

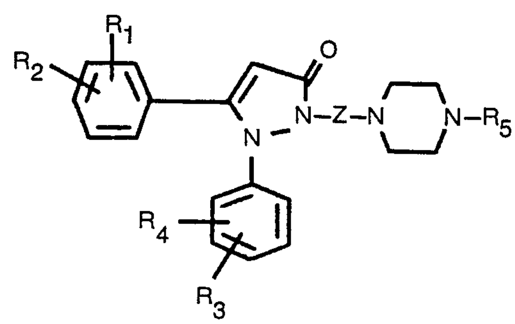

I

worin $R_1$, $R_2$, $R_3$, $R_4$, $R_5$ und Z die in Anspruch 1 angegebene Bedeutung besitzen sowie deren Säureadditionssalzen, dadurch gekennzeichnet, daß man

a) Verbindungen der Formel II oder III

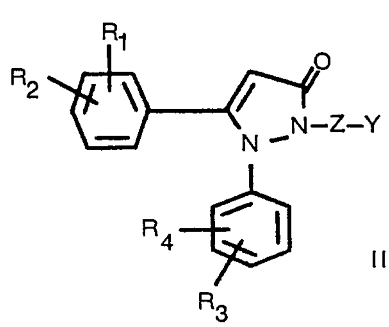

II

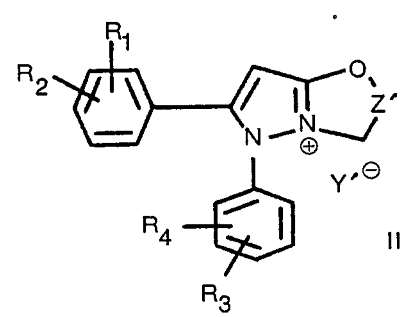

III

worin $R_1$, $R_2$, $R_3$ und $R_4$ obige Bedeutung besitzen, und Z obige Bedeutung besitzt und Y einen aminolytisch abspaltbaren Rest bedeutet oder Z' eine Alkylenkette mit 2—4 Kohlenstoffatomen und Y' Halogen bedeuten, oder deren Gemische mit einer Verbindung der Formel V

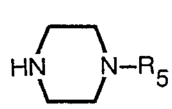

V

worin $R_5$ obige Bedeutung besitzt, umsetzt, oder

b) zur Herstellung von Verbindungen der Formel Ia

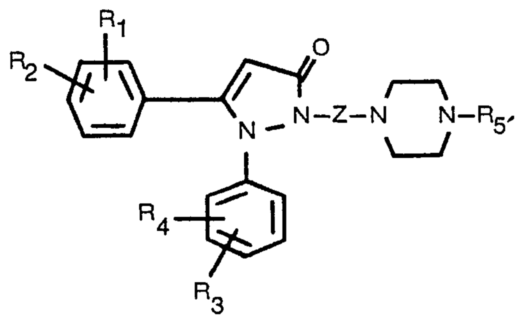

Ia

worin $R_1$, $R_2$, $R_3$, $R_4$ und Z obige Bedeutung besitzen, und $R_5'$ für eine substituierte Phenylgruppe a' steht

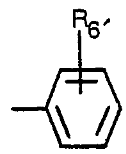

a'

worin $R_6'$ ortho- oder paraständig ist und $CF_3$ bedeutet, Verbindungen der Formel IV

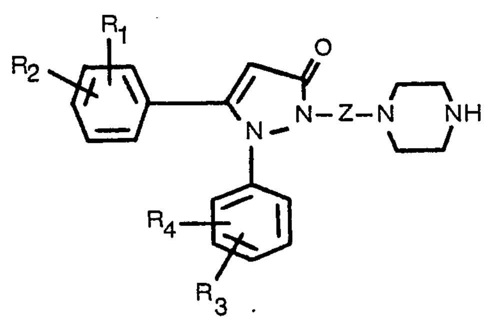

IV

worin $R_1$, $R_2$, $R_3$, $R_4$ und Z obige Bedeutung besitzen, mit Verbindungen der Formel VI

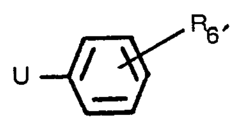

VI

worin $R_6'$ obige Bedeutung besitzt und U Halogen bedeutet, umsetzt, und gegebenenfalls freie Verbindungen der Formel I in ihre Säureadditionssalze überführt oder die Säureadditionssalze in die freien Verbindungen der Formel I überführt.

9. Heilmittel, enthaltend eine pharmakologisch wirksame Menge einer 1,5-Diphenyl-pyrazolon-Verbindung gemäß Anspruch 1 und übliche pharmazeutische Hilfs- und/oder Trägerstoffe.

**Patentansprüche für den Vertragsstaat: AT**

1. Verfahren zur Herstellung von 1,5-Diphenylpyrazolin-3-on-Verbindungen der allgemeinen Formel I

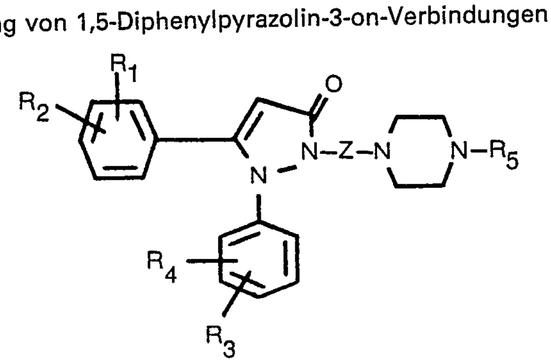

I

worin

$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet,

$R_2$ Wasserstoff, Alkyl mit 1—4 Kohlenstoffatomen, Alkoxy mit 1—4 Kohlenstoffatomen oder Halogen bedeutet, oder

$R_1$ und $R_2$ an benachbarte Kohlenstoffatome gebunden sind und zusammen Methylendioxy oder Äthylendioxy bedeuten,

$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_4$ Wasserstoff, Alkyl mit 1—4 Kohlenstoffatomen, Alkoxy mit 1—4 Kohlenstoffatomen oder Halogen bedeutet, oder

$R_3$ und $R_4$ an benachbarte Kohlenstoffatome gebunden sind und zusammen Methylendioxy oder Äthylendioxy bedeuten,

Z eine Alkylengruppe mit 2—6 Kohlenstoffatomen bedeutet,

$R_5$ Pyridyl, welches unsubstituiert oder durch Alkyl mit 1—4 Kohlenstoffatomen, Halogen oder Alkoxy mit 1—4 Kohlenstoffatomen monosubstituiert ist, oder Thienyl oder eine gegebenenfalls substituierte Phenylgruppe a bedeutet

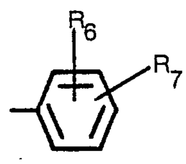

a

worin

$R_6$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_7$ Wasserstoff, Alkyl mit 1—4 Kohlenstoffatomen, Alkoxy mit 1—4 Kohlenstoffatomen oder Halogen bedeutet, oder

$R_6$ und $R_7$ an benachbarte Kohlenstoffatome gebunden sind und zusammen Methylendioxy oder Äthylendioxy bedeuten,

sowie deren Säureadditionssalze, dadurch gekennzeichnet, daß man

a) Verbindungen der Formel II oder III

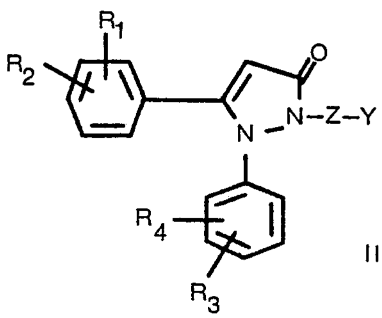

II

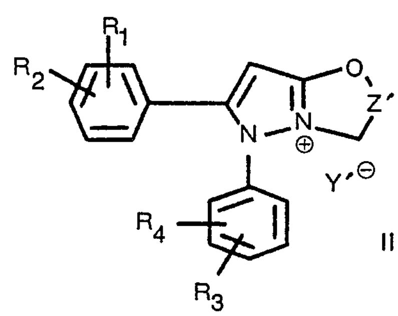

III

worin $R_1$, $R_2$, $R_3$ und $R_4$ obige Bedeutung besitzen, und Z obige Bedeutung besitzt und Y einen aminolytisch abspaltbaren Rest bedeutet oder Z′ eine Alkylenkette mit 2—4 Kohlenstoffatomen und Y′ Halogen bedeuten, oder deren Gemische mit einer Verbindung der Formel V

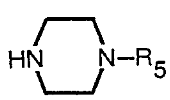

V

worin $R_5$ obige Bedeutung besitzt, umsetzt, oder

b) zur Herstellung von Verbindungen der Formel Ia

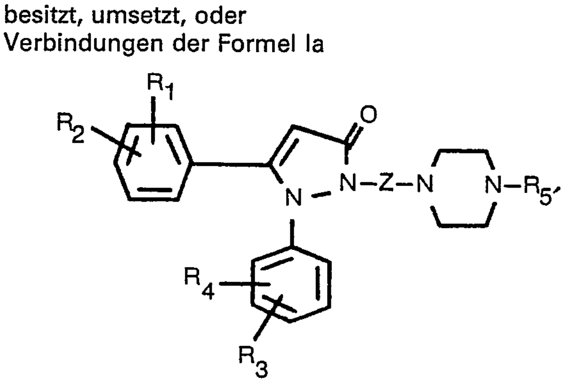

Ia

worin $R_1$, $R_2$, $R_3$, $R_4$ und Z obige Bedeutung besitzen, und $R_5'$ für eine substituierte Phenylgruppe a' steht

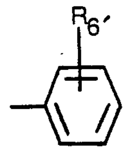

a'

worin $R_6'$ ortho- oder paraständig ist und $CF_3$ bedeutet, Verbindungen der Formel IV

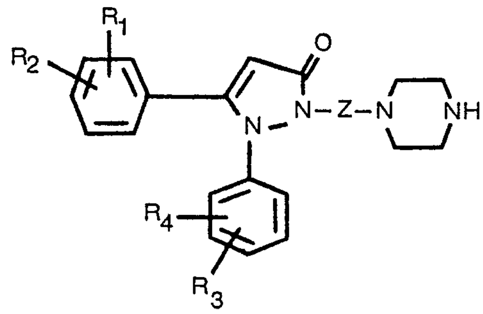

IV

worin $R_1$, $R_2$, $R_3$, $R_4$ und Z obige Bedeutung besitzen, mit Verbindungen der Formel VI

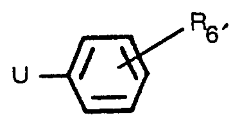

VI

worin $R_6'$ obige Bedeutung besitzt und U Halogen bedeutet, umsetzt, und gegebenenfalls freie Verbindungen der Formel I in ihre Säureadditionssalze überführt oder die Säureadditionssalze in die freien Verbindungen der Formel I überführt.

2. Verfahren nach Anspruch 1, dadurch gekennzeichnet, daß man 1,5-Diphenylpyrazolin-3-on-Verbindungen der Formel I herstellt, worin Z, $R_1$, $R_2$, $R_3$ und $R_4$ obige Bedeutung besitzen und $R_5$ für eine gegebenenfalls substituierte Phenylgruppe a steht

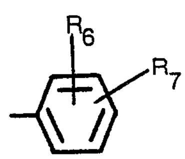

a

worin $R_6$ und $R_7$ obige Bedeutung besitzen.

3. Verfahren nach Anspruch 1, dadurch gekennzeichnet, daß man 1,5-Diphenylpyrazolin-3-on-Verbindungen der Formel I herstellt, worin Z, $R_1$, $R_2$, $R_3$ und $R_4$ obige Bedeutung besitzen und $R_5$ Pyridyl, welches unsubstituiert oder durch Alkyl mit 1—4 Kohlenstoffatomen, Halogen oder Alkoxy mit 1—4 Kohlenstoffatomen monosubstituiert ist, bedeutet.

4. Verfahren nach Anspruch 3, dadurch gekennzeichnet, daß man 1,5-Diphenyl-2-{3-[4-(2-pyridyl)-piperazin-1-yl]-propyl}-pyrazolin-3-on und dessen Säureadditionssalze herstellt.

5. Verfahren zur Herstellung von Verbindungen der allgemeinen Formel II oder III

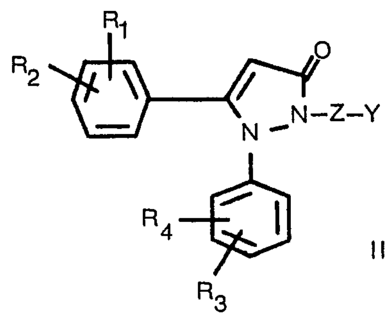

II

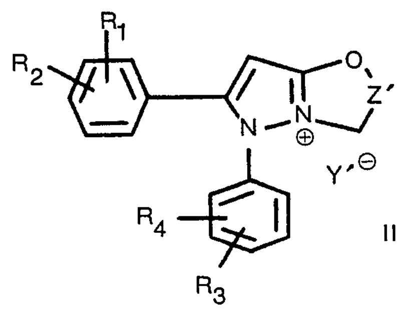

III

worin

$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_2$ Wasserstoff, Alkyl mit 1—4 Kohlenstoffatomen, Alkoxy mit 1—4 Kohlenstoffatomen oder Halogen bedeutet, oder

$R_1$ und $R_2$ an benachbarte Kohlenstoffatome gebunden sind und zusammen Methylendioxy oder Äthylendioxy bedeuten,

$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_4$ Wasserstoff, Alkyl mit 1—4 Kohlenstoffatomen, Alkoxy mit 1—4 Kohlenstoffatomen oder Halogen bedeutet, oder

$R_3$ und $R_4$ an benachbarte Kohlenstoffatome gebunden sind und zusammen Methylendioxy oder Äthylendioxy bedeuten, und

Z eine Alkylengruppe mit 2—6 Kohlenstoffatomen und

Y einen aminolytisch abspaltbaren Rest bedeuten, oder

Z' eine Alkylenkette mit 2—4 Kohlenstoffatomen und

Y' Halogen bedeuten,

und deren Gemischen, dadurch gekennzeichnet, daß man Alkalimetallsalze von 1,5-Diphenylpyrazolonverbindungen der Formel VIII

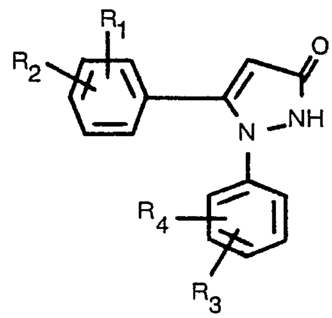

VIII

worin $R_1$, $R_2$, $R_3$ und $R_4$ obige Bedeutung besitzen, mit Verbindungen der Formel VII

$$Y—Z—Y' \qquad \text{VII}$$

worin Y, Y' und Z obige Bedeutung besitzen, umsetzt, wobei für den Fall, daß Y Halogen und Z eine Alkylenkette mit 2—4 Kohlenstoffatomen bedeutet, neben Verbindungen der Formel II auch Verbindungen der Formel III erhalten werden.

6. Verfahren zur Herstellung von Verbindungen der allgemeinen Formel IV

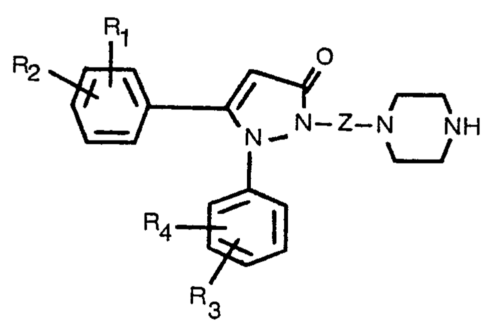

IV

worin

$R_1$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_2$ Wasserstoff, Alkyl mit 1—4 Kohlenstoffatomen, Alkoxy mit 1—4 Kohlenstoffatomen oder Halogen bedeutet, oder

$R_1$ und $R_2$ an benachbarte Kohlenstoffatome gebunden sind und zusammen Methylendioxy oder Äthylendioxy bedeuten,

$R_3$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet, und

$R_4$ Wasserstoff, Alkyl mit 1—4 Kohlenstoffatomen, Alkoxy mit 1—4 Kohlenstoffatomen oder Halogen bedeutet, oder

$R_3$ und $R_4$ an benachbarte Kohlenstoffatome gebunden sind und zusammen Methylendioxy oder Äthylendioxy bedeuten, und

Z eine Alkylengruppe mit 2—6 Kohlenstoffatomen bedeutet,

und deren Säureadditionssalzen, dadurch gekennzeichnet, daß man

a) Verbindungen der Formel II oder III

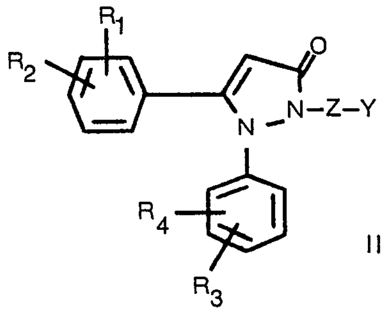

II

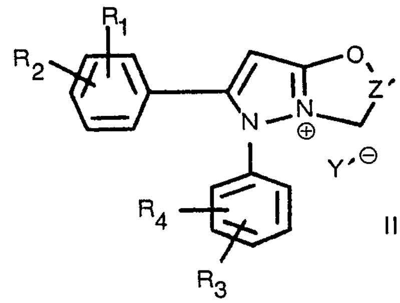

III

worin $R_1$, $R_2$, $R_3$ und $R_4$ obige Bedeutung besitzen, und Z obige Bedeutung besitzt und Y einen aminolytisch abspaltbaren Rest bedeutet oder Z' eine Alkylenkette mit 2—4 Kohlenstoffatomen und Y' Halogen bedeuten, oder deren Gemische mit einem Überschuß Piperazin umsetzt oder

b) aus Verbindungen der Formel IX

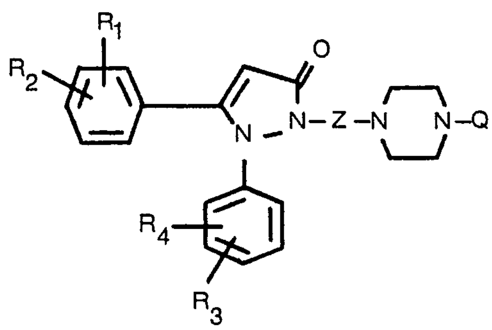

IX

worin $R_1$, $R_2$, $R_3$, $R_4$ und Z obige Bedeutung besitzen, und Q für eine Aminschutzgruppe steht, die Aminschutzgruppe abspaltet.

**Revendications pour les Etats contractants: BE CH DE FR GB IT LU NL SE**

1. 1,5-diphénylpyrazoline-3-ones de formule générale I

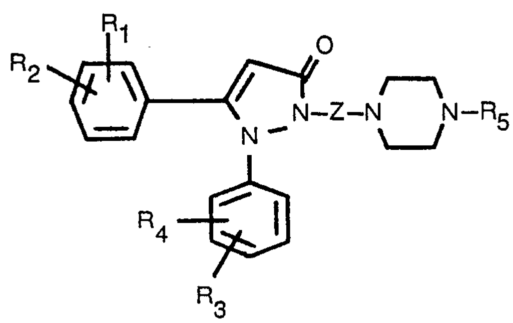

I

dans laquelle

$R_1$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy comportant 1 à 4 atomes de carbone, et

$R_2$ représente un atome d'hydrogène, un groupe alkyle ayant 1 à 4 atomes de carbone, alcoxy ayant 1 à 4 atomes de carbone ou halogéno, ou bien

$R_1$ et $R_2$ sont fixés sur des atomes de carbone voisins et forment ensemble un groupe méthylènedioxy ou éthylènedioxy,

$R_3$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_4$ représente un atome d'hydrogène, un groupe alkyle ayant 1 à 4 atomes de carbone, alcoxy ayant 1 à 4 atomes de carbone ou halogéno, ou bien

$R_3$ et $R_4$ sont fixés sur des atomes de carbone voisins et forment ensemble un groupe méthylènedioxy ou éthylènedioxy,

Z représente un groupe alkylène ayant 2 à 6 atomes de carbone,

$R_5$ représente un groupe pyridyle (non substitué ou qui est monosubstitué par un groupe alkyle ayant 1 à 4 atomes de carbone, halogéno ou alcoxy ayant 1 à 4 atomes de carbone), ou un groupe thiényle ou un groupe phényle a, éventuellement substitué:

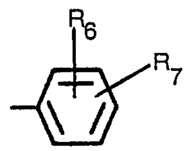

a

dans lequel

$R_6$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_7$ représente un atome d'hydrogène, un groupe alkyle ayant 1 à 4 atomes de carbone, alcoxy ayant 1 à 4 atomes de carbone ou halogéno, ou bien

$R_6$ et $R_7$ sont fixés sur des atomes de carbone voisins et forment ensemble un groupe méthylènedioxy ou éthylènedioxy,

ainsi que leurs sels d'addition d'acides.

2. 1,5-diphénylpyrazoline-3-ones selon la revendication 1, dans lesquelles Z, $R_1$, $R_2$, $R_3$ et $R_4$ ont le sens précité et $R_5$ représente un groupe phényle a, éventuellement substitué

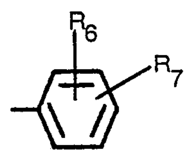

a

dans lequel $R_6$ et $R_7$ ont le sens ci-dessus.

3. 1,5-diphénylpyrazoline-3-ones selon la revendication 1, dans lesquelles Z, $R_1$, $R_2$, $R_3$ et $R_4$ ont le sens précité, et $R_5$ représente un groupe pyridyle, qui n'est pas substitué ou bien est monosubstitué par un groupe alkyle comportant 1 à 4 atomes de carbone, halogéno ou alcoxy ayant 1 à 4 atomes de carbone.

4. La 1,5-diphényl-2-{3-[4-(2-pyridyl)-pipérazine-1-yl]-propyl}-pyrazoline-3-one, et ses sels d'addition d'acides, selon la revendication 3.

5. Composés de formules générales II ou III

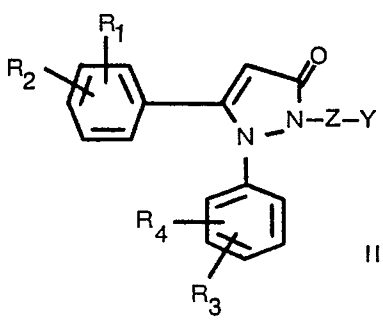

II.

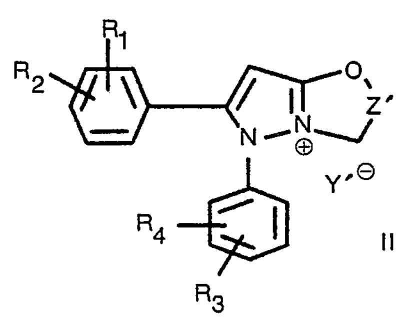

III

dans lesquelles

$R_1$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_2$ représente un atome d'hydrogène, un groupe alkyle ayant 1 à 4 atomes de carbone, alcoxy ayant 1 à 4 atomes de carbone ou halogéno, ou bien

$R_1$ et $R_2$ sont fixés sur des atomes de carbone voisins et forment ensemble un groupe méthylènedioxy ou éthylènedioxy,

$R_3$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_4$ représente un atome d'hydrogène, un groupe alkyle ayant 1 à 4 atomes de carbone, alcoxy ayant 1 à 4 atomes de carbone ou halogéno, ou bien

$R_3$ et $R_4$ sont fixés sur des atomes de carbone voisins et forment ensemble un groupe méthylènedioxy ou éthylènedioxy, et

Z représente un groupe alkylène ayant 2 à 6 atomes de carbone, et

Y représente un reste pouvant être séparé par aminolyse, ou bien

Z' représente une chaîne alkylène ayant 1 à 4 atomes de carbone, et

Y' représente un atome d'halogène,
et leurs mélanges.

6. Composés de formule II selon la revendication 5, dans laquelle $R_1$, $R_2$, $R_3$, $R_4$ et Z ont le sens précité et Y représente un halogène.

7. Composés de formule générale IV

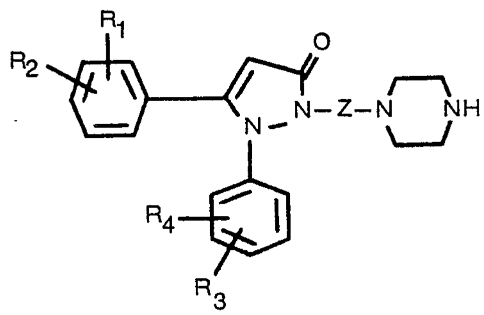

IV

dans laquelle

$R_1$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_2$ représente un atome d'hydrogène, un groupe alkyle ayant 1 à 4 atomes de carbone, alcoxy ayant 1 à 4 atomes de carbone ou halogéno, ou bien

$R_1$ et $R_2$ sont fixés sur des atomes de carbone voisins et ils forment ensemble un groupe méthylènedioxy ou éthylènedioxy,

$R_3$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_4$ représente un atome d'hydrogène, un groupe alkyle ayant 1 à 4 atomes de carbone, alcoxy ayant 1 à 4 atomes de carbone ou halogéno, ou bien

$R_3$ et $R_4$ sont fixés sur des atomes de carbone voisins et ils forment ensemble un groupe méthylènedioxy ou éthylènedioxy, et

Z représente un groupe alkylène ayant 2 à 6 atomes de carbone,

ou leurs sels d'addition d'acides.

8. Procédé pour préparer les 1,5-diphénylpyrazoline-3-ones de formule générale I

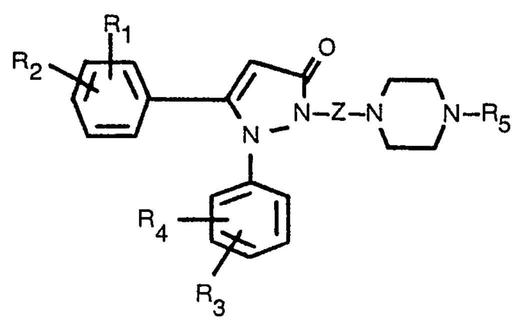

I

dans laquelle $R_1$, $R_2$, $R_3$, $R_4$, $R_5$ et Z ont le sens indiqué à la revendication 1, ainsi que leurs sels d'addition d'acides, procédé caractérisé en ce que

a) on fait réagir des composés de formules II ou III:

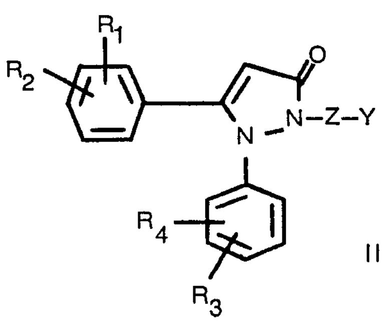

II

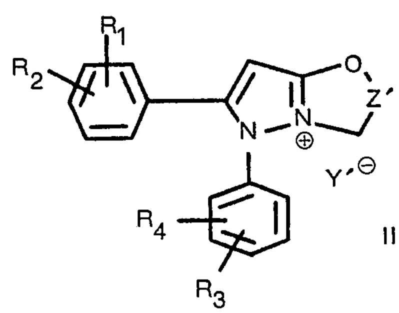

III

dans lesquelles

$R_1$, $R_2$, $R_3$, $R_4$ ont le sens précité, et Z a le sens précité, et Y représente un reste pouvant être séparé par aminolyse ou bien Z' représente une chaîne alkylène ayant 2 à 4 atomes de carbone et Y' représente un atome d'halogène, ou leurs mélanges, avec un composé de formule V

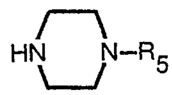
V

dans laquelle

$R_5$ a le sens précité, ou bein

b) pour préparer les composés de formule Ia:

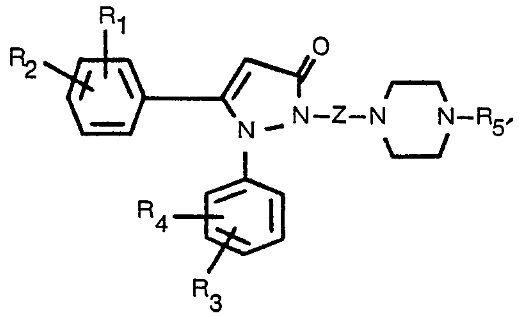
Ia

dans laquelle

$R_1$, $R_2$, $R_3$, $R_4$ et Z ont le sens précité, et $R_{5'}$ représente un groupe phényle a' substitué:

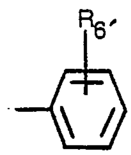
a'

dans lequel

$R_6'$ est fixé en ortho ou en para et représente un groupe $CF_3$,

on fait réagir des composés de formule IV

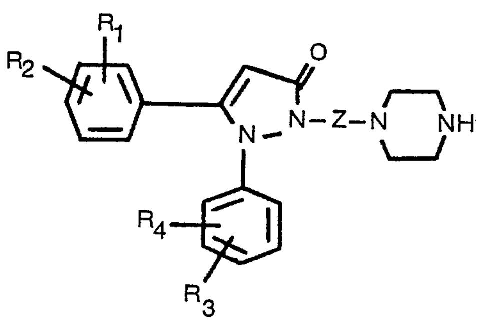
IV

dans laquelle

$R_1$, $R_2$, $R_3$, $R_4$ et Z ont le sens précité, avec des composés de formule VI

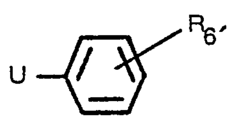
VI

dans laquelle

$R_6'$ a le sens précité et U représente un atome d'halogène,

et, éventuellement, on transforme les composés libres de formule I en leurs sels d'addition d'acides, ou bien on transforme les sels d'addition d'acides en les composés libres de formule I.

9. Médicament, contenant une quantité pharmacologiquement active d'une 1,5-diphényl-pyrazolone selon la revendication 1 et les adjuvants, excipients et/ou supports ou véhicules pharmaceutiques usuels.

**Revendications pour l'Etat contractant: AT**

1. Procédé de préparation de 1,5-diphénylpyrazoline-3-ones de formule générale I

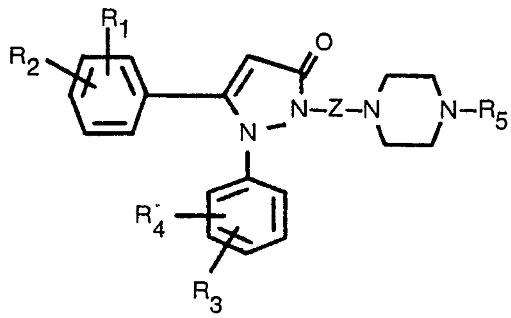

I

dans laquelle

$R_1$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy comportant 1 à 4 atomes de carbone, et

$R_2$ représente un atome d'hydrogène, un groupe alkyle ayant 1 à 4 atomes de carbone, alcoxy ayant 1 à 4 atomes de carbone ou halogéno, ou bien

$R_1$ et $R_2$ sont fixés sur des atomes de carbone voisins et forment ensemble un groupe méthylènedioxy ou éthylènedioxy,

$R_3$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_4$ représente un atome d'hydrogène, un groupe alkyle ayant 1 à 4 atomes de carbone, alcoxy ayant 1 à 4 atomes de carbone ou halogéno, ou bien

$R_3$ et $R_4$ sont fixés sur des atomes de carbone voisins et forment ensemble un groupe méthylènedioxy ou éthylènedioxy,

Z représente un groupe alkylène ayant 2 à 6 atomes de carbone,

$R_5$ représente un groupe pyridyle (non substitué ou qui est monosubstitué par un groupe alkyle ayant 1 à 4 atomes de carbone, halogéno ou alcoxy ayant 1 à 4 atomes de carbone), ou un groupe thiényle ou un groupe phényle a, éventuellement substitué:

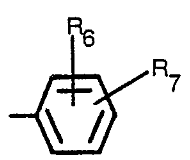

a

dans lequel

$R_6$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_7$ représente un atome d'hydrogène, un groupe alkyle ayant 1 à 4 atomes de carbone, alcoxy ayant 1 à 4 atomes de carbone ou halogéno, ou bien

$R_6$ et $R_7$ sont fixés sur des atomes de carbone voisins et forment ensemble un groupe méthylènedioxy ou éthylènedioxy,

ainsi que leurs sels d'addition d'acides, procédé caractérisé en ce que

a) on fait réagir des composés de formules II ou III:

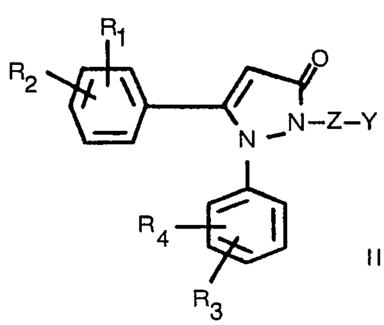

II

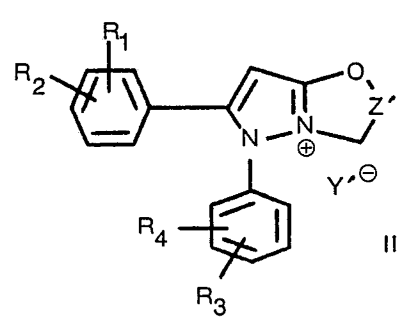

III

dans lesquelles

$R_1$, $R_2$, $R_3$, $R_4$ ont le sens précité, et Z a le sens précité, et Y représente un reste pouvant être séparé par aminolyse ou bien Z' représente une chaîne alkylène ayant 2 à 4 atomes de carbone et Y' représente un atome d'halogène, ou leurs mélanges, avec un composé de formule V

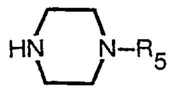

V

dans laquelle

$R_5$ a le sens précité, ou bein

b) pour préparer les composés de formule Ia:

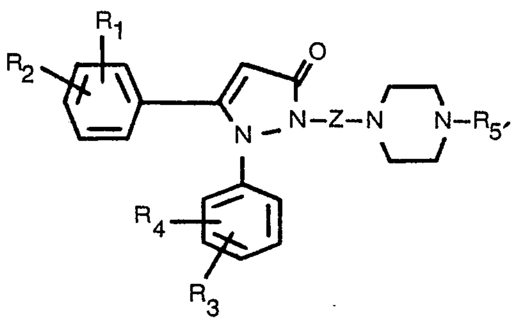

Ia

dans laquelle

$R_1$, $R_2$, $R_3$, $R_4$ et Z ont le sens précité, et $R_{5'}$ représente un groupe phényle a' substitué:

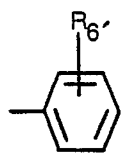

a'

dans lequel

$R_6'$ est fixé en ortho ou en para et représente un groupe $CF_3$,

on fait réagir des composés de formule IV

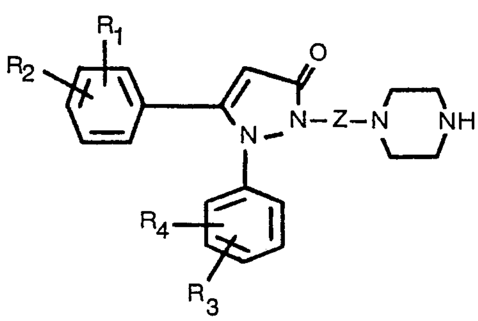

IV

dans laquelle

$R_1$, $R_2$, $R_3$, $R_4$ et Z ont le sens précité, avec des composés de formule VI

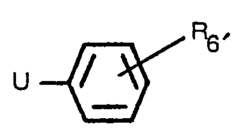

VI

dans laquelle

$R_6'$ a le sens précité et U représente un atome d'halogène,

et, éventuellement, on transforme les composés libres de formule I en leurs sels d'addition d'acide, ou bien on transforme les sels d'addition d'acides en les composés libres de formule I.

2. Procédé selon la revendication 1, caractérisé en ce qu'on prépare des 1,5-diphénylpyrazoline-3-ones de formule I, dans laquelle Z, $R_1$, $R_2$, $R_3$ et $R_4$ ont le sens précité et $R_5$ représente un groupe phényle a, éventuellement substitué

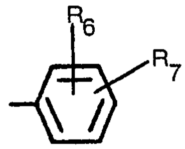

a

dans lequel $R_6$ et $R_7$ ont le sens précité.

3. Procédé selon la revendication 1, caractérisé en ce qu'on prépare des 1,5-diphénylpyrazoline-3-ones de formule I, dans laquelle Z, $R_1$, $R_2$, $R_3$ et $R_4$ ont le sens précité et $R_5$ représente un groupe pyridyle, qui n'est pas substitué ou est monosubstitué par un groupe alkyle ayant 1 à 4 atomes de carbone, par un halogène ou par un groupe alcoxy ayant 1 à 4 atomes de carbone.

4. Procédé selon la revendication 3, caractérisé en ce qu'on prépare la 1,5-diphényl-2-{3-[4-(2-pyridyl)-pipérazine-1-yl]-propyl}-pyrazoline-3-one et ses sels d'addition d'acides.

5. Procédé de préparation de composés répondant à la formule générale II ou III,

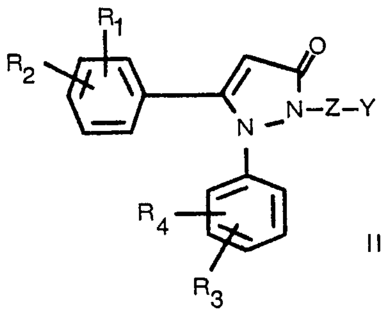

II

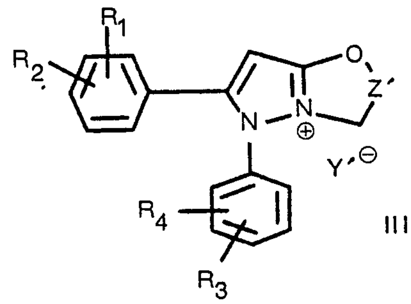

III

dans laquelle

$R_1$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_2$ représente un atome d'hydrogène, un groupe alkyle ayant 1 à 4 atomes de carbone, alcoxy ayant 1 à 4 atomes de carbone ou halogéno, ou bien

$R_1$ et $R_2$ sont fixés sur des atomes de carbone voisins et forment ensemble un groupe méthylènedioxy ou éthylènedioxy,

$R_3$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_4$ représente un atome d'hydrogène, un groupe alkyle ayant 1 à 4 atomes de carbone, alcoxy ayant 1 à 4 atomes de carbone ou halogéno, ou bien

$R_3$ et $R_4$ sont fixés sur des atomes de carbone voisins et forment ensemble un groupe méthylènedioxy ou éthylènedioxy, et

Z représente un groupe alkylène ayant 2 à 6 atomes de carbone, et

Y représente un reste pouvant être séparé par aminolyse, ou bien

Z' représente une chaîne alkylène ayant 2 à 4 atomes de carbone, et

Y' représente un atome d'halogène,

et leurs mélanges, procédé caractérisé en ce qu'on fait réagir des sels de métaux alcalins de 1,5-diphénylpyrazolones de formule VIII

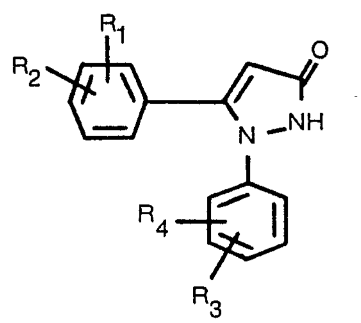

VIII

dans laquelle

$R_1$, $R_2$, $R_3$ et $R_4$ ont le sens précité, avec des composés de formule VII

Y—Z—Y' VII

dans laquelle Y, Y' et Z ont le sens précité, et, si Y représente un halogène et Z une chaîne alkylène ayant 2 à 4 atomes de carbone, on obtient, en plus des composés de formule II, également des composés de formule III.

6. Procédé pour préparer des composés de formule générale IV:

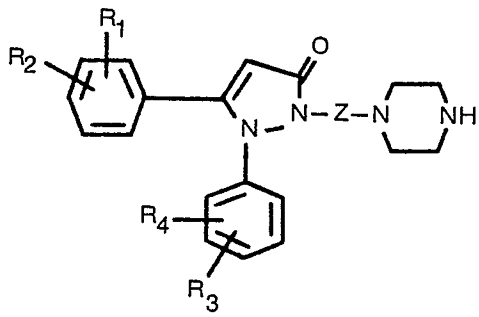

IV

dans laquelle

$R_1$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_2$ représente un atome d'hydrogène, un groupe alkyle ayant 1 à 4 atomes de carbone, alcoxy ayant 1 à 4 atomes de carbone ou halogéno, ou bien

$R_1$ et $R_2$ sont fixés sur des atomes de carbone voisins et ils forment ensemble un groupe méthylènedioxy ou éthylènedioxy,

$R_3$ représente un atome d'hydrogène, un groupe alkyle ayant 1 à 4 atomes de carbone, alcoxy ayant 1 à 4 atomes de carbone, hydroxyle, halogéno, trifluorométhyle ou alcanoyloxy ayant 1 à 4 atomes de carbone, et

$R_4$ représente un atome d'hydrogène, un groupe alkyle ayant 1 à 4 atomes de carbone, alcoxy ayant 1 à 4 atomes de carbone ou halogéno, ou bien

$R_3$ et $R_4$ sont fixés sur des atomes de carbone voisins et ils forment ensemble un groupe méthylènedioxy ou éthylènedioxy, et

Z représente un groupe alkylène ayant 2 à 6 atomes de carbone,

ou leurs sels d'addition d'acides,

caractérisé en ce que:

a) on fait réagir des composés de formules II ou III:

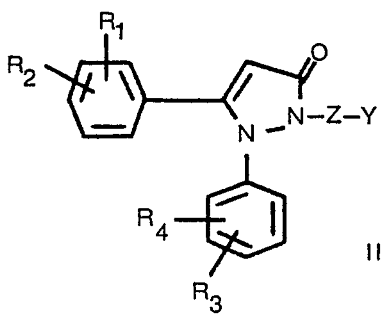

II

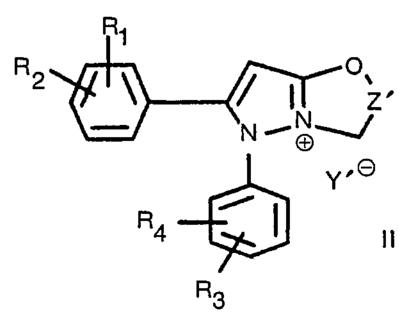

III

dans lesquelles

$R_1$, $R_2$, $R_3$, $R_4$ ont le sens précité, et Z a le sens précité, et Y représente un reste pouvant être séparé par aminolyse ou bien Z' représente une chaîne alkylène ayant 2 à 4 atomes de carbone et Y' un atome d'halogène, ou leurs mélanges, avec un excès de pipérazine, ou bien

b) on sépare le groupe protecteur de fonction amine de composés de formule IX

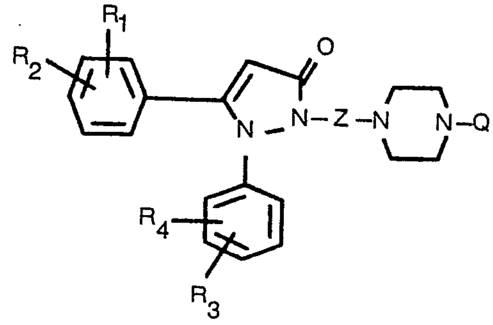

IX

dans laquelle

$R_1$, $R_2$, $R_3$, $R_4$ et Z ont le sens précité, et Q représente un groupe protecteur de fonction amine.

**Claims for the Contracting States: BE CH DE FR GB IT LU NL SE**

1. 1,5-diphenylpyrazolin-3-one compounds of the general Formula I,

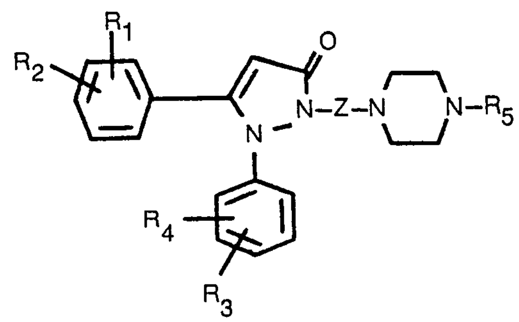

I

wherein

$R_1$ is hydrogen, alkyl with 1—4 carbon atoms, alkoxy with 1—4 carbon atoms, hydroxy, halogen, trifluoromethyl or alkanoyloxy with 1—4 carbon atoms, and

$R_2$ is hydrogen, alkyl with 1—4 carbon atoms, alkoxy with 1—4 carbon atoms or halogen, or

$R_1$ and $R_2$ are bonded to adjacent carbon atoms and together represent methylene dioxy or ethylene dioxy,

$R_3$ is hydrogen, alkyl with 1—4 carbon atoms, alkoxy with 1—4 carbon atoms, hydroxy, halogen, trifluoromethyl or alkanoyloxy with 1—4 carbon atoms, and

$R_4$ is hydrogen, alkyl with 1—4 carbon atoms, alkoxy with 1—4 carbon atoms or halogen, or

$R_3$ and $R_4$ are bonded to adjacent carbon atoms and together represent methylene dioxy or ethylene dioxy,

Z is an alkylene group with 2—6 carbon atoms,

$R_5$ is pyridyl which is un-substituted or mono-substituted by alkyl with 1—4 carbon atoms, halogen or alkoxy with 1—4 carbon atoms, or thienyl or an optionally substituted phenyl group a,

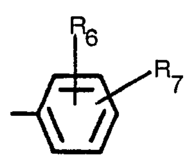

a

wherein

$R_6$ is hydrogen, alkyl with 1—4 carbon atoms, alkoxy with 1—4 carbon atoms, hydroxy, halogen, trifluoromethyl or alkanoyloxy with 1—4 carbon atoms, and

$R_7$ is hydrogen, alkyl with 1—4 carbon atoms, alkoxy with 1—4 carbon atoms or halogen, or

$R_6$ and $R_7$ are bonded to adjacent carbon atoms and together represent methylene dioxy or ethylene dioxy,

and their acid addition salts.

2. 1,5-diphenylpyrazolin-3-one compounds according to Claim 1, wherein Z, $R_1$, $R_2$, $R_3$ and $R_4$ have the above meanings and $R_5$ stands for an optionally substituted phenyl group a,

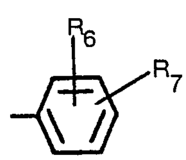

a

wherein $R_6$ and $R_7$ have the above meanings.

3. 1,5-diphenylpyrazolin-3-one compounds according to Claim 1, wherein Z, $R_1$, $R_2$, $R_3$ and $R_4$ have the above meanings and $R_5$ is pyridyl, which is un-substituted or mono-substituted by alkyl with 1—4 carbon atoms, halogen or alkoxy with 1—4 carbon atoms.

4. 1,5-diphenyl-2-{3-[4-(2-pyridyl)-piperazin-1-yl]-propyl}-pyrazolin-3-one and its acid addition salts according to Claim 3.

5. Compounds of the general formula II or III,

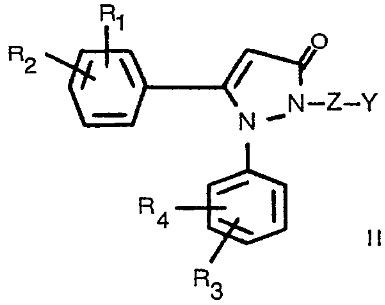

II

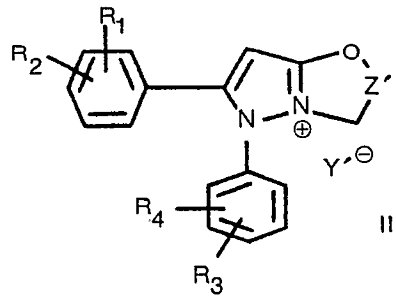

III

wherein

$R_1$ is hydrogen, alkyl with 1—4 carbon atoms, alkoxy with 1—4 carbon atoms, hydroxy, halogen, trifluoromethyl or alkanoyloxy with 1—4 carbon atoms, and

$R_2$ is hydrogen, alkyl with 1—4 carbon atoms, alkoxy with 1—4 carbon atoms or halogen, or

$R_1$ and $R_2$ are bonded to adjacent carbon atoms and together represent methylene dioxy or ethylene dioxy,

$R_3$ is hydrogen, alkyl with 1—4 carbon atoms, alkoxy with 1—4 carbon atoms, hydroxy, halogen, trifluoromethyl or alkanoyloxy with 1—4 carbon atoms, and

$R_4$ is hydrogen, alkyl with 1—4 carbon atoms, alkoxy with 1—4 carbon atoms or halogen,

$R_3$ and $R_4$ are bonded to adjacent carbon atoms and together represent methylene dioxy or ethylene dioxy, and

Z is an alkylene group with 2—6 carbon atoms and

Y is an aminolytically cleavable radical, or

Z' is an alkylene chain with 1—4 carbon atoms and

Y' is halogen,

and their mixtures.

6. Compounds of Formula II according to Claim 5, wherein $R_1$, $R_2$, $R_3$, $R_4$ and Z have the above meanings and Y is halogen.

7. Compounds of general Formula IV,

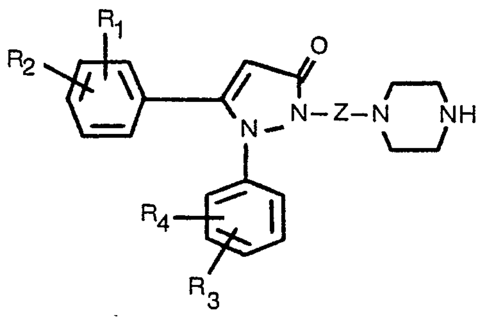

IV

wherein

$R_1$ is hydrogen, alkyl with 1—4 carbon atoms, alkoxy with 1—4 carbon atoms, hydroxy, halogen, trifluoromethyl or alkanoyloxy with 1—4 carbon atoms, and

$R_2$ is hydrogen, alkyl with 1—4 carbon atoms, alkoxy with 1—4 carbon atoms or halogen, or

$R_1$ and $R_2$ are bonded to adjacent carbon atoms and together represent methylene dioxy or ethylene dioxy,

$R_3$ is hydrogen, alkyl with 1—4 carbon atoms, alkoxy with 1—4 carbon atoms, hydroxy, halogen, trifluoromethyl or alkanoyloxy with 1—4 carbon atoms, and

$R_4$ is hydrogen, alkyl with 1—4 carbon atoms, alkoxy with 1—4 carbon atoms or halogen, or

$R_3$ and $R_4$ are bonded to adjacent carbon atoms and together represent methylene dioxy or ethylene dioxy, and

Z is an alkylene group with 2—6 carbon atoms,

and their acid addition salts.

8. Method for producing 1,5-diphenylpyrazolin-3-one compounds of the general Formula I,

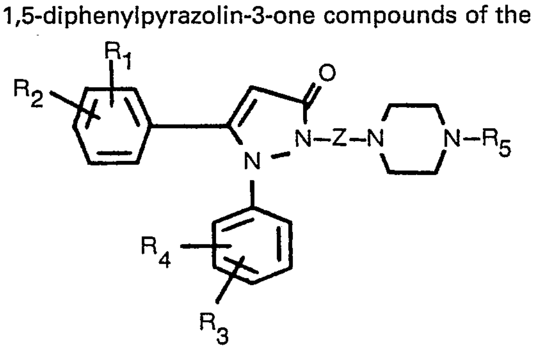

I

wherein $R_1$, $R_2$, $R_3$, $R_4$, $R_5$ and Z have the meanings given in Claim 1, and their acid addition salts, characterised in that

a) compounds of Formula II or III,

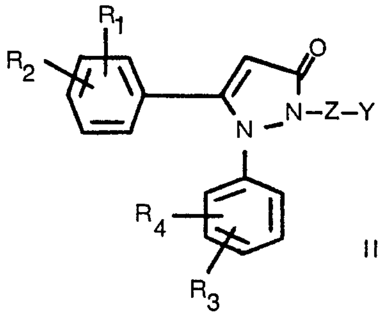

II

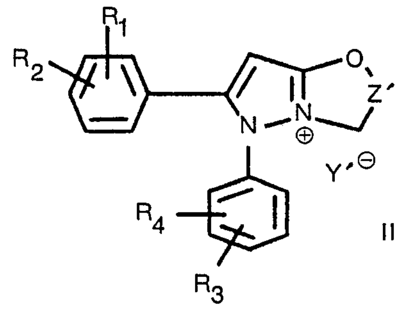

III

wherein

$R_1$, $R_2$, $R_3$ and $R_4$ have the above meanings and Z has the above meaning, and Y is an aminolytically cleavable radical, or Z′ is an alkylene chain with 2—4 carbon atoms and Y′ is halogen, or mixtures thereof are reacted with a compound of Formula V,

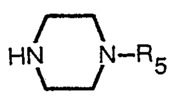

V

wherein

$R_5$ has the above meaning, or

b) to produce compounds of Formula Ia,

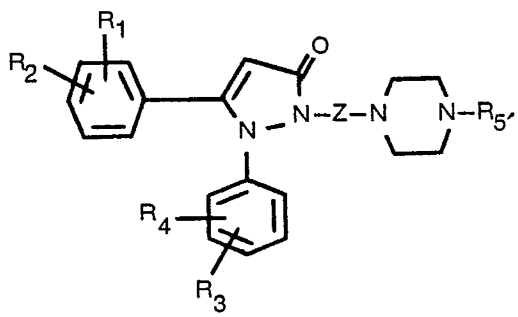

Ia

wherein

$R_1$, $R_2$, $R_3$, $R_4$ and Z have the above meanings and $R_5'$ stands for a substituted phenyl group a′,

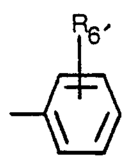

a′

wherein

$R_6'$ is in the ortho- or para-position and is $CF_3$, compounds of Formula IV,

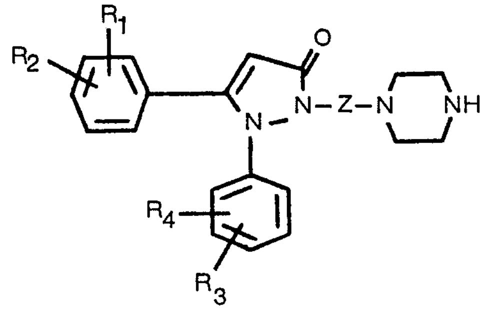

IV

wherein

$R_1$, $R_2$, $R_3$, $R_4$ and Z have the above meanings, are reacted with compounds of Formula VI,

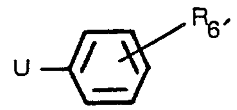

VI

wherein

$R_6'$ has the above meaning and U is halogen, and optionally free compounds of Formula I are converted into their acid addition salts or the acid addition salts are converted into the free compounds of Formula I.

9. Medicament containing a pharmacologically effective quantity of a 1,5-diphenyl-pyrazolone compound according to Claim 1 and conventional pharmaceutical auxiliaries and/or carriers.

**Claims for the Contracting State: AT**

1. Process for producing 1,5-diphenylpyrazolin-3-one compounds of the general Formula I,

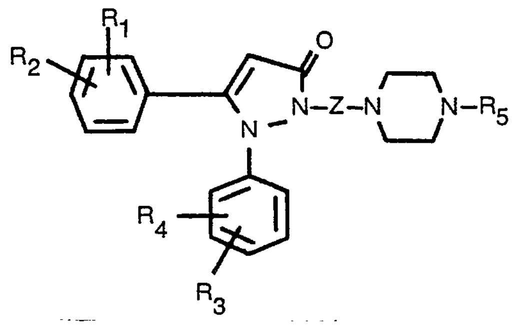

I

wherein

$R_1$ is hydrogen, alkyl with 1—4 carbon atoms, alkoxy with 1—4 carbon atoms, hydroxy, halogen, trifluoromethyl or alkanoyloxy with 1—4 carbon atoms, and

$R_2$ is hydrogen, alkyl with 1—4 carbon atoms, alkoxy with 1—4 carbon atoms or halogen, or

$R_1$ and $R_2$ are bonded to adjacent carbon atoms and together represent methylene dioxy or ethylene dioxy,

$R_3$ is hydrogen, alkyl with 1—4 carbon atoms, alkoxy with 1—4 carbon atoms, hydroxy, halogen, trifluoromethyl or alkanoyloxy with 1—4 carbon atoms, and

$R_4$ is hydrogen, alkyl with 1—4 carbon atoms, alkoxy with 1—4 carbon atoms or halogen, or

$R_3$ and $R_4$ are bonded to adjacent carbon atoms and together represent methylene dioxy or ethylene dioxy,

Z is an alkylene group with 2—6 carbon atoms,

$R_5$ is pyridyl which is un-substituted or mono-substituted by alkyl with 1—4 carbon atoms, halogen or alkoxy with 1—4 carbon atoms, or thienyl or an optionally substituted phenyl group a,

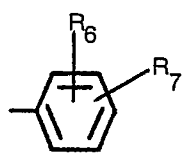

a

wherein

$R_6$ is hydrogen, alkyl with 1—4 carbon atoms, alkoxy with 1—4 carbon atoms, hydroxy, halogen, trifluoromethyl or alkanoyloxy with 1—4 carbon atoms, and

$R_7$ is hydrogen, alkyl with 1—4 carbon atoms, alkoxy with 1—4 carbon atoms or halogen, or

$R_6$ and $R_7$ are bonded to adjacent carbon atoms and together represent methylene dioxy or ethylene dioxy,

and their acid addition salts, characterised in that

a) compounds of Formula II or III,

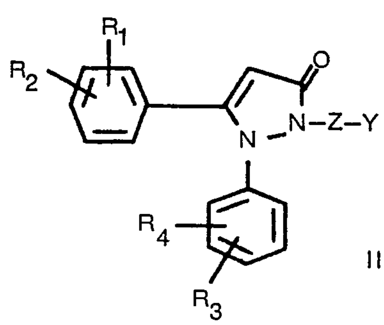

II

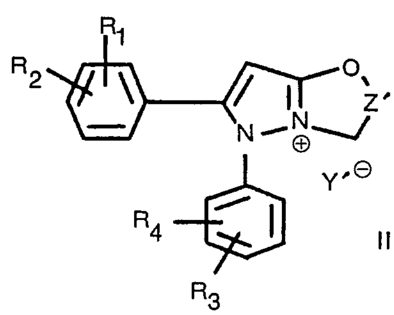

III

wherein

$R_1$, $R_2$, $R_3$ and $R_4$ have the above meanings and Z has the above meaning, and Y is an aminolytically cleavable radical, or Z' is an alkylene chain with 2—4 carbon atoms and Y' is halogen, or mixtures thereof are reacted with a compound of Formula V,

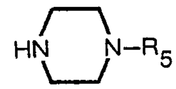

V

wherein

$R_5$ has the above meaning, or

b) to produce compounds of Formula Ia,

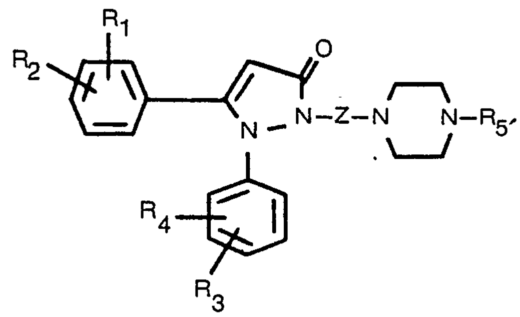

Ia

wherein

$R_1$, $R_2$, $R_3$, $R_4$ and Z have the above meanings and $R_5'$ stands for a substituted phenyl group a',

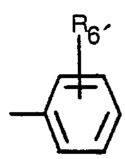

a'

wherein

$R_6'$ is in the ortho- or para-position and is $CF_3$, compounds of Formula IV,

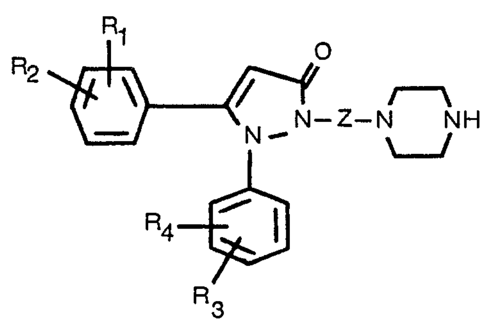

IV

wherein

$R_1$, $R_2$, $R_3$, $R_4$ and Z have the above meanings, are reacted with compounds of Formula VI,

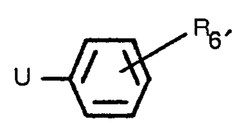

VI

wherein

$R_6'$ has the above meaning and U is halogen, and optionally free compounds of Formula I are converted into their acid addition salts or the acid addition salts are converted into the free compounds of Formula I.

2. Process according to Claim 1, characterised in that 1,5-diphenylpyrazolin-3-one compounds of Formula I are produced wherein Z, $R_1$, $R_2$, $R_3$ and $R_4$ have the above meanings and $R_5$ stands for an optionally substituted phenyl group a,

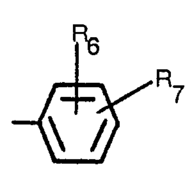

a

wherein $R_6$ and $R_7$ have the above meanings.

3. Process according to Claim 1, characterised in that 1,5-diphenylpyrazolin-3-one compounds of Formula I are produced wherein Z, $R_1$, $R_2$, $R_3$ and $R_4$ have the above meanings and $R_5$ is pyridyl, which is unsubstituted or mono-substituted by alkyl with 1—4 carbon atoms, halogen or alkoxy with 1—4 carbon atoms.

4. Process according to Claim 3, characterised in that 1,5-diphenyl-2-{3-[4-(2-pyridyl)-piperazin-1-yl]-propyl}-pyrazolin-3-one and its acid addition salts are produced.

5. Process for producing compounds of the general Formula II or III,

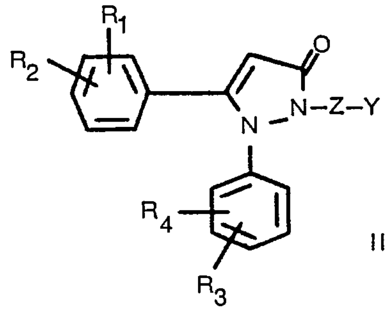
II

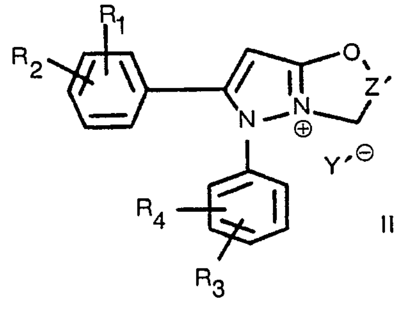
III

wherein

$R_1$ is hydrogen, alkyl with 1—4 carbon atoms, alkoxy with 1—4 carbon atoms, hydroxy, halogen, trifluoromethyl or alkanoyloxy with 1—4 carbon atoms, and

$R_2$ is hydrogen, alkyl with 1—4 carbon atoms, alkoxy with 1—4 carbon atoms or halogen, or

$R_1$ and $R_2$ are bonded to adjacent carbon atoms and together represent methylene dioxy or ethylene dioxy,

$R_3$ is hydrogen, alkyl with 1—4 carbon atoms, alkoxy with 1—4 carbon atoms, hydroxy, halogen, trifluoromethyl or alkanoyloxy with 1—4 carbon atoms, and

$R_4$ is hydrogen, alkyl with 1—4 carbon atoms, alkoxy with 1—4 carbon atoms or halogen, or

$R_3$ and $R_4$ are bonded to adjacent carbon atoms and together represent methylene dioxy or ethylene dioxy, and

Z is an alkylene group with 2—6 carbon atoms and

Y is an aminolytically cleavable radical, or

Z' is an alkylene chain with 2—4 carbon atoms and

Y' is halogen,

and their mixtures, characterised in that alkali metal salts of 1,5-diphenylpyrazolone compounds of Formula VIII,

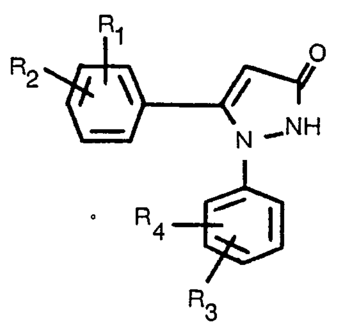
VIII

wherein

$R_1$, $R_2$, $R_3$ and $R_4$ have the above meanings, are reacted with compounds of Formula VII,

$$Y—Z—Y' \qquad VII$$

wherein Y, Y' and Z have the above meanings, whereby if Y is halogen and Z an alkylene chain with 2—4 carbon atoms, compounds of Formula III as well as compounds of Formula II are obtained.

6. Process for producing compounds of general Formula IV,

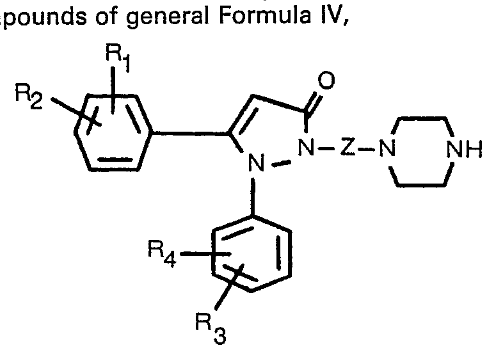
IV

wherein

$R_1$ is hydrogen, alkyl with 1—4 carbon atoms, alkoxy with 1—4 carbon atoms, hydroxy, halogen, trifluoromethyl or alkanoyloxy with 1—4 carbon atoms, and

$R_2$ is hydrogen, alkyl with 1—4 carbon atoms, alkoxy with 1—4 carbon atoms or halogen, or

$R_1$ and $R_2$ are bonded to adjacent carbon atoms and together represent methylene dioxy or ethylene dioxy, and

$R_3$ is hydrogen, alkyl with 1—4 carbon atoms, alkoxy with 1—4 carbon atoms, hydroxy, halogen, trifluoromethyl or alkanoyloxy with 1—4 carbon atoms, and

$R_4$ is hydrogen, alkyl with 1—4 carbon atoms, alkoxy with 1—4 carbon atoms or halogen, or

$R_3$ and $R_4$ are bonded to adjacent carbon atoms and together represent methylene dioxy or ethylene dioxy,

Z is an alkylene group with 2—6 carbon atoms,

and their acid addition salts, characterised in that

a) compounds of Formula II or III,

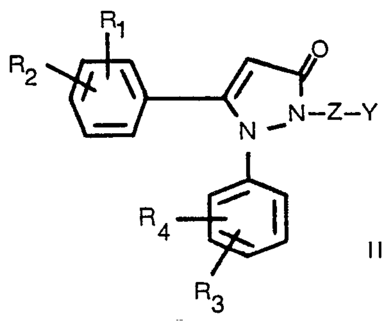
II

,

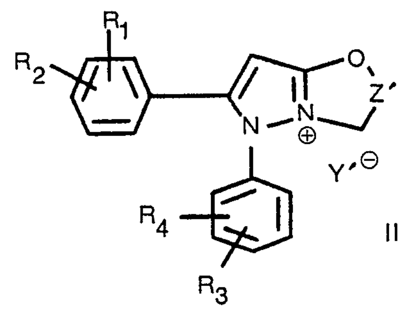
III

wherein

$R_1$, $R_2$, $R_3$ and $R_4$ have the above meanings and Z has the above meaning, and Y is an aminolytically cleavable radical, or Z' is an alkylene chain with 2—4 carbon atoms and Y' is halogen, or mixtures thereof are reacted with an excess of piperazine or

b) the protective amine group is cleaved off from compounds of Formula IX,

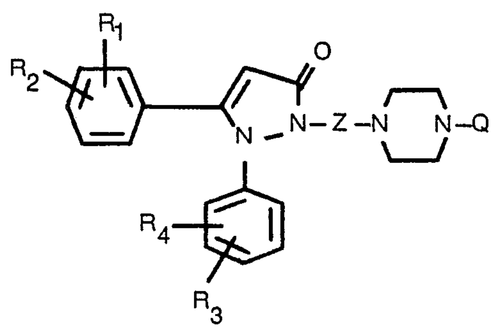
IX

wherein

$R_1$, $R_2$, $R_3$, $R_4$ and Z have the above meanings and Q stands for a protective amine group.